Europäisches Patentamt

European Patent Office

Office européen des brevets

(11) Publication number: **0 412 116 B1**

# EUROPEAN PATENT SPECIFICATION

(45) Date of publication of patent specification: **29.11.95**  (51) Int. Cl.⁶: **C12Q  1/68**, G01N 33/574, C12N 15/00

(21) Application number: **89905846.5**

(22) Date of filing: **18.04.89**

(86) International application number: **PCT/US89/01636**

(87) International publication number: **WO 89/10412 (02.11.89 89/26)**

The file contains technical information submitted after the application was filed and not included in this specification

(54) **DETECTION OF NEU GENE EXPRESSION AND PRODUCTS.**

(30) Priority: **18.04.88 US 182501**
**13.01.89 US 297188**

(43) Date of publication of application:
**13.02.91 Bulletin  91/07**

(45) Publication of the grant of the patent:
**29.11.95 Bulletin  95/48**

(84) Designated Contracting States:
**AT BE CH DE FR GB IT LI LU NL SE**

(56) References cited:
**EP-A- 108 564**
**WO-A-87/07646**

**CHEMICAL ABSTRACTS, vol. 106, 1987, Columbus, OH (US); J.A. DREBIN et al., p. 385, no. 31140g/**

(73) Proprietor: **APPLIED BIOTECHNOLOGY, INC.**
**80 Rogers Street**
**Cambridge**
**Massachusetts 02142 (US)**

Proprietor: **WHITEHEAD INSTITUTE FOR BIOMEDICAL RESEARCH**
**Nine Cambridge Center**
**Cambridge, MA 02142 (US)**

(72) Inventor: **WEINBERG, Robert, A.**
**25 Copley Street**
**Brookline, MA 02146 (US)**
Inventor: **MAZZARA, Gail, P.**
**10 Manchester Road**
**Winchester, MA 02890 (US)**
Inventor: **MORGAN, Jonathan, H.**
**3 Arrowhead Lane**
**Franklin, MA 02038 (US)**

CHEMICAL ABSTRACTS, vol. 108, 1988, Columbus, OH (US); J.A. DREBIN et al., p. 532, no. 184829e/

SCIENCE, vol. 235, 09 January 1987; D.J. SLAMON et al., pp. 177-182/

CHEMICAL ABSTRACTS, vol. 107, 1987, Columbus, OH (US); M. VAN DE VIJVER et al., p. 177, no. 1863y/

Inventor: McKENZIE, Sara, J.
201 Ocean Street
Lynn, MA 01902 (US)
Inventor: MARKS, Paula, J.
93 Evans Road
Brookline, MA 02146 (US)

(74) Representative: Holdcroft, James Gerald, Dr. et al
Graham Watt & Co.,
Riverhead
Sevenoaks, Kent TN13 2BN (GB)

**Description**

Background

An increasing body of evidence implicates somatic mutations as causally important in the induction of human cancers. These somatic mutations may accumulate in the genomes of previously normal cells, some of which may then demonstrate the phenotypes associated with malignant growth. Such oncogenic mutations may include a number of different types of alterations in DNA structure, including deletions, translocations, amplifications and single nucleotide alterations. The latter, also known as point mutations, may frequently intervene in carcinogenesis, in that a variety of mutagenic chemicals induce such mutations. In addition, such mutations may occur spontaneously as a result of mistakes in DNA replication.

Point mutations have been directly implicated in the causation of 10-15% of human tumors. These tumors carry oncogenes of the ras gene family, which differ from their normal cellular counterpart proto-oncogenes by the presence of a point mutation at one of several sites in these genes. These mutations represent qualitative changes in the tumor cell genome which distinguish these cells from normal cells and provide a basis for diagnosis of the genetic origin of a tumor under study. Identification of the mutations that have created active oncogenes may provide important diagnostic and prognostic clues for tumor development. For example, a number of mutations have been found to alter the 12th codon of the ras oncogenes, causing replacement of glycine, which is a normally present, by any of a number of alternative amino acid residues. Such amino acid substitutions do not have equivalent effects; some substitutions (e.g., valine) create a potent transforming allele while others (e.g., proline) have only a limited effect on cellular phenotype. Thus, the identity of a particular nucleotide substitution may be a strong determinant of the behavior of the tumor cell (e.g., its rate of growth, invasiveness, etc.). As a result, DNA probes of oncogene mutations have promise as diagnostic reagents in clinical oncology.

Such probes are useful, however, only if a region of a gene of interest in which point mutations are likely to occur has been identified. Unless identification of such a region has been made, it is impractical to use oligonucleotide probes of limited size (e.g., 10-20 nucleotides long) to scan an entire gene, which might well be 30,000 or more base pairs long; for example, if a 15-nucleotide probe were used to scan the entire length of a gene 33,000 base pairs long, 3000-5000 separate probes would be required.

Thus, although DNA probes of oncogene mutations have potential as diagnostic tools, they cannot be used effectively unless a discrete region of mutation in the gene, which is causally related to activation of the gene's oncogenic function, has been identified. Without such localization of the region of mutational activation, use of DNA probes specific for point mutations is impractical.

The amplification and/or overexpression of proto-oncogenes have also been implicated in the causation of human tumors. The c-myc and N-myc proto-oncogene are amplified in small cell carcinoma of the lung and neuroblastoma, respectively. The N-myc proto-oncogene was found to be amplified 3-300 times in many stage II, III and IV tumors. The presence of the amplification of the N-myc proto-oncogene is always accompanied by an increased expression of the gene product though not necessarily proportional to the extent of amplification. More importantly the number of N-myc gene copies in primary untreated neuroblastomas is a clinically important prognostic factor that is independent of stage (Seeger et al., N Engl J Med., 1985; 313:1111-6). There is a significant correlation between genomic amplification and rapid tumor progression. Thus, it is clear that detection of the amplification of a proto-oncogene or expression of its mRNA by nucleic acid hybridization techniques or detection of overexpression of an oncogene's protein product by immunological methods could have great prognostic value.

In addition, the following references are disclosed as background:

International Publication No. WO 87/07646, published December 17, 1987, discloses antibodies specific for gene products encoded by neu oncogenes.

Drebin, et al., Proc. Nat. Acad. Sci. (USA) 83:9129-9133 (1986) describe the use of a monoclonal antibody (7.16.4) reactive with a cell surface domain of the rat neu gene encoded p185 for inhibition of tumor growth.

Drebin, et al., Oncogene 2:273-277 (1988) describe antibodies reactive with distinctive domains of the neu oncogene encoded p185 molecule and their antitumor effects in mice.

Slamon, et al., Science 235:177-235 (1987) describe amplification of the HER-2/neu gene in human breast cancer cell lines.

Van de Vijver, et al., Molecular and Cellular Biology 7:2019-2023 (1987) describe amplification of the neu(c-erb-B2) oncogene in human mammary tumors and amplification of the linked c-erbA oncogene.

Disclosure of the Invention

This invention relates to an immunoassay for detecting the overexpression of human neu gene encoded p185 protein in a sample of cells comprising:

(a) obtaining a lysate of the sample of cells;

(b) contacting the lysate with an antibody specific for the extracellular domain of the human neu gene encoded p185 protein under conditions suitable for binding; and

(c) comparing the level of binding with the level of binding in a lysate of normal cells, wherein a higher level of binding is indicative of the overexpression of the human neu gene encoded p185 protein in the sample of cells.

It has been determined that the rat neu proto-oncogene, which encodes a protein resembling a growth factor receptor, is converted into an oncogene by a single nucleotide alteration or point mutation. This point mutation was initially seen in a rat neuroblastoma induced by transplacental exposure to a carcinogen (e.g., ethylnitrosourea) and found to affect the amino acid sequence of the transmembrane region of the p185 encoded by the DNA. That is, a valine present in the normal protein is replaced by a glutamic acid residue.

Nucleic acid probes used to assay for similar point mutations suspected to be present in seven additional neu oncogenes, each of which arose in a separate, independently induced tumor, demonstrated the presence of the same activating mutations in all seven neu oncogenes. The same amino acid substitution (glutamic acid replacing valine) resulted in these cells. Further assays with nucleic acid probes homologous to the neu gene demonstrates that the mutagen methylnitrosourea induces formation of nervous system tumors and activation of neu genes at the same position as shown to occur in the ethylnitrosurea-induced tumors.

The human homolog of the neu gene (also known as c-erbB2 or HER2) may achieve an oncogenic state through the action of a similar mechanism: alteration of a single nucleotide in the normal cellular DNA sequence (the proto-oncogene), resulting in activation of the oncogene. Due to sequence differences between the rat and human neu alleles it is not possible to generate the same mutation (valine to glutamic acid) with a single base change. This valine to glutamic acid substitution requires two base changes which makes it a very statistically unlikely event. The effect of single base pair changes at this position in the human neu allele generating substitutions of amino acids other than glutamic acid is unknown but could be expected to occur.

Alternatively, the oncogenic activity of the neu proto-oncogene may be activated by gene amplification or other events that lead to the overexpression of the neu proto-oncogene.

The activating lesion found can be identified in the DNA of a variety of spontaneously arising human tumors through the use of nucleic acid probes constructed to be specifically reactive with the region of the human neu gene corresponding to the region in the rat neu oncogene known to contain the activating mutations. Identification in human tumor cells of the activating point mutation responsible for conversion of the proto-oncogene into the neu oncogene can serve as the basis for construction of nucleic acid hybridization probes useful in testing human tumor DNAs for the presence or absence of point mutations responsible for activation of neu oncogenes. These hybridization probes can be used in detecting the occurrence of the neu proto-oncogene and of the neu oncogene in cells and in determining the profile of oncogene activations in human tumor specimens. Antibodies specific for the p185 protein encoded by the neu oncogene, which can be used to detect the occurrence of the neu oncogene, or proto-oncogene, are described. Particular "capture" immunoassays are described for detection of the human neu antigen in biological fluids, such as human serum, plasma or urine or in normal, preneoplastic or neoplastic cells.

Brief Description of the Drawings

Figure 1 is a schematic representation of pSV2neu, created by inserting the neu gene cDNA indicated into the pSV2 expression vector.

Figure 2 presents electrophoretic gel patterns characteristic of cell lines containing pSV2neu constructions.

Figure 3 shows nucleotides 1968 to 2073 and the predicted amino acid sequence for the normal rat neu gene and the transforming rat neu gene.

Figure 4 shows the nucleotide sequence of nucleic acid probes corresponding in sequence to a) the wild type (normal), b) the mutant neu version of the neu gene, c) DNA from DHFR G8, and d) a modified version of the mutant neu gene in which there is a T to G transversion.

Figure 5 shows electrophoretic gel patterns after hybridization of DNA from neu transfectants with nucleic acid probes.

Figure 6 shows electrophoretic gel patterns after hybridization of DNA from tumor cell lines and normal BDIX DNA with oligonucleotide probes.

Figure 7 shows electrophoretic gel patterns after probing of fourteen transfectants containing ten independent activated neu genes with either the oligonucleotide corresponding to the normal gene (proto-oncogene) (top) or the oligonucleotide corresponding to the transforming gene (oncogene) (bottom)

Figure 8 is a schematic representation of a full length neu cDNA clone constructed from cDNAs of two cervical carcinoma cell lines (ME180 and SW1710).

Figure 9 is a schematic representation of two human neu vectors, pMax neu and pMax delta neu, constructed by inserting the neu gene cDNA indicated into the pMax expression vector, and the pMax-Sph expression vector, respectively.

Figure 10 is a schematic representation of a hybridization probe created by inserting the portion of the neu gene indicated into the pGEM expression vector.

Figure 11 is a schematic representation of plasmid vector pLJdelta neu, created by inserting the neu gene cDNA indicated into the pLJdelta expression vector.

Figure 12 shows results of a capture immunoassay in which lysates from a variety of human breast carcinoma cell lines were tested for the presence of the neu antigen using the capture ELISA system. An anti-neu monoclonal antibody (TA-1) was used to capture the p185 neu antigen and the anti-neu monoclonal antibody NA-3 labeled with biotin was used as the detection reagent.

Figure 13 shows results (microgram of tumor lysate vs. optical density) of a comparison between tumor lysate of a nude mouse tumor expressing neu (X-3-5) and tumor lysate of a neu negative tumor (3T3/ras), an anti-neu monoclonal antibody (NB-3) specific for the p185 neu antigen.

Figure 14 shows the results of an assay of cell lysates prepared from a normal piece of human breast tissue (2747-01-050) or a breat carcinoma (2427-01-050) and tested for the presence of neu antigen using the capture format. The assay was performed with an anti-neu monoclonal antibody designated TA-1 as the capture reagent and the monoclonal antibody BD-5 as the biotin-labeled detection reagent.

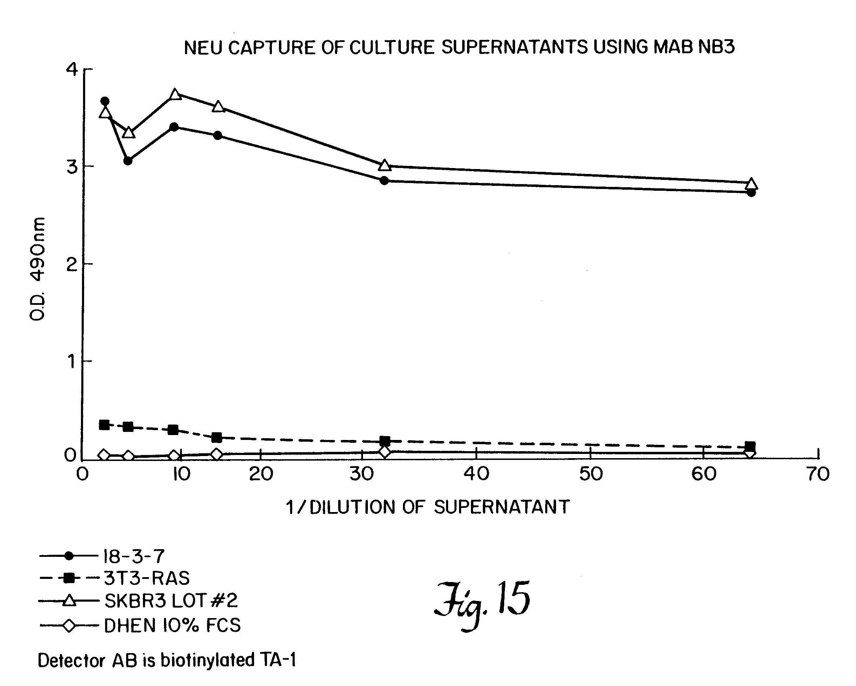

Figure 15 shoes results of analysis of supernatant fluids from 18-3-7 cells (NIH3T3 cells transformed with the human neu gene and expressing the p185 protein on the cell surface), 3T3 ras cells (NIH3T3 cells transformed with the ras gene and not expressing the human p185 protein on the cell surface), and SK-BR-3 human breast carcinoma cells and culture media-DMEM supplemented with 10% fetal calf serum. In the capture immunoassay, an anti-neu monoclonal antibody, NB-3, was used as the capture reagent and an anti-neu monoclonal antibody, TA-1, labeled with biotin was used as the detection reagent.

Figure 16 shows results of a capture immunoassay using mice in which normal mouse sera, (T144), sera from mice bearing tumors expressing the p185 protein (18-3-7 mouse), and from mice bearing tumors not expressing the p185 protein (3T3 (ras)) were assayed using an anti-neu monoclonal antibody, TA-1, as the capture antibody and an anti-neu monoclonal antibody, BD-5, labeled with biotin as the detection reagent.

Figure 17 shows the results of a capture immunoassay in which the anti-neu monoclonal antibody TA-1 was used as the capture reagent and BD-5 labeled with biotin was used as the detection reagent. Samples for analysis included normal human plasma and plasma from two breast carcinoma patients.

Figure 18 is a graphic representation of the average value of neu oncogene (units of neu) in plasma from three sets of human patients.

## Detailed Description of the Invention

The neu oncogene was originally isolated from a rat neuroblastoma induced by transplacental exposure to the carcinogen ethylnitrosourea. The activating event was a single point mutation which caused a valine of the proto-oncogene to be replaced by a glutamic acid residue. The human homologue of the neu oncogene has also been cloned and its oncogenic potential can be activated by making the same mutation found in the activated rat allele, although two adjacent point mutations are required. In addition, the human allele is oncogenic when over-expressed without any mutational changes. This is in direct contrast to the rat neu allele which when not mutated is non-transforming at any level of expression. A third mechanism may activate the human neu allele, truncation. When the amino terminal portion of the human neu proto-oncogene is deleted it then becomes transforming.

The involvement of proto-oncogenes in the genesis of human cancers is well documented. The mechanism by which proto-oncogenes contribute to the malignant process is varied. These mechanisms include alterations of the proto-oncogene's primary sequence or control of expression. Alteration of control of expression can be accomplished by increased expression of the pre-existing gene or by increasing the number of copies of the gene (gene amplification). Determination of the oncogene involved and the method of activation may have significant implications for diagnosis, prognosis and therapy.

Recently, the neu proto-oncogene has been shown to be amplified in human breast cancers. Neu was amplified from 2- to greater than 20 times in 30% of breast tumors. The presence of neu amplification was a significant predictor of both overall survival time and time to relapse (Slamon, et al., Science 1987; 235:177-182).

Mutational Activation

It is now known that cellular genes other than the ras genes can be converted into oncogenes by point mutations. It has recently been shown that the rat neu proto-oncogene, which encodes a protein resembling a growth factor receptor, can also be converted into an oncogene by a single nucleotide alteration. Thus, it has been shown that the neu proto-oncogene (i.e., the nucleotide sequence present in the normal genome of normal non-tumor cells) can undergo mutational activation into its corresponding oncogene (i.e., the nucleotide sequence whose expression within a cell causes its conversion from a normal cell into a tumor cell) by a single nucleotide substitution. This point mutation, initially demonstrated in a rat neuroblastoma induced by transplacental exposure to ethylnitrosourea, has also been shown to occur in seven other neu oncogenes, each of which arose in a separate exposure, independently induced by ethylnitrosourea. Detection of the point mutations relied on the use of nucleic acid probes specific for the region initially shown to contain the activating mutation. In all cases, the same amino acid substitution resulted: a valine normally present in the transmembrane region of the encoded p185 protein was replaced by a glutamic acid residue. This finding suggests that nucleotide substitution at only a limited number of sites in the neu proto-oncogene will result in its becoming an active oncogene.

Transfectants which arose from DNAs from ten methylnitrosourea-induced nervous system tumors which contained activated neu genes have also been shown to have the identical alteration in their DNA and are presumed to have the same sequence change as that present in the ethylnitrosourea-induced tumors, based on their differential hybridization to an oligonucleotide probe corresponding to the transforming gene. Thus, nucleic acid probes homologous to the neu gene have been shown to be able to detect activated neu genes in tumors induced by exposure to methylnitrosourea. In addition, this demonstrates that both mutagens cause activation of neu genes at the same position and that activated neu genes can arise in both BDIX rats (ethylnitrosourea induced tumors) and Buffalo rats (methylnitrosourea- induced tumors).

Human versions of the rat neu genes have been isolated and are also referred to as c-erbB2 or HER2. Yamamoto, T. et al., Nature, 319:230-234 (1986); Coussens, L. et al., Science, 230:1132-1139 (1985). The DNA sequences or both the rat and the human clones predict a 1260 amino acid protein product of the neu gene.

Mutational activation of the human homologs of the rat neu genes, referred to here as the human neu oncogenes, may occur through a similar mechanism: single nucleotide substitution or point mutation in the human neu gene. It is highly likely that the single nucleotide substitution occurs in the region of the human neu proto-oncogene that corresponds with the region in the rat neu oncogene shown to contain the activating mutations. It is possible to determine the presence or absence of the activating lesion in the DNA of a variety of spontaneously arising human tumors through the use of nucleic acid probes specifically reactive either with regions unique to the neu oncogene or with regions unique to the corresponding neu proto-oncogene. That is, hybridization probes can be constructed which will react (hybridize) with a nucleotide sequence occurring in either a neu oncogene or its corresponding proto-oncogene (but not in both).

Such probes can be used to test human tumor DNAs for the occurrence of a point mutation responsible for activation of the neu oncogene. For example, radiolabelled nucleic acid probes can be used in the Southern blot hybridization procedure to detect activation of the neu oncogene in human tumors of clinical interest.

Amplification/Overexpression

Amplification of the human neu proto-oncogene may be another mechanism for activating the oncogenic potential of the human neu proto-oncogene. The detection of the amplification of the human neu proto-oncogene can be accomplished using hybridization probes derived from any portion of the neu gene. These probes do not need to distinguish between the oncogene and proto-oncogene. Such methods have been used successfully to detect the amplification of the human N-myc gene in neuroblastomas.

Amplification of human oncogenes have been found in several tumor types. The best documented is the amplification of the N-myc gene in neuroblastomas. This gene was found to be amplified 3-300 times in many stage II, III and IV tumors. More importantly the number of N-myc copies in primary untreated neuroblastomas is a clinically important prognostic factor that is independent of stage (Seeger et al., N. Engl. J. Med. 1985; 313: 1111-6). There is a significant correlation between genomic amplification and rapid tumor progression. There is also some evidence that overexpression of the N-myc gene in the absence of gene amplification has a similar correlation with prognosis. The amplification of oncogenes has been detected in a variety of other tumors. These include retinoblastoma (N-myc), acute promyelocytic leukemia, carcinomas of the colon, breast, lung, and stomach (c-myc) as well as many others.

Detecting amplification of the neu gene and/or overexpression of the neu gene product can be used to assay for the presence of cancer in mammalian cells. The amplification or overexpression is measured at the protein level.

The p185 proteins encoded by neu oncogenes are different from those encoded by their corresponding proto-oncogenes, therefore it is also possible to develop serological reagents, such as polyclonal antibodies or monoclonal antibodies, which are specific for the altered or the normal amino acid sequences in such proteins. These reagents can be used to provide highly sensitive tests for the presence or absence of neu oncogenes by detecting the occurence of the nutated or altered gene products they encode.

With serological reagents, the neu gene encoded protein can be detected using serological reagents on the surface of cells by immunohistochemical techniques and in various biological fluids using immunoassay techniques. For these tests, antibodies specific for the amino acid sequence, or a portion of the amino acid sequence of the neu encoded produce are employed.

Human tissue specimens (e.g., biopsy samples) can be tested for expression of the neu gene by immunohistochemical techniques such as the immunoperoxidase staining procedure. Alternatively, immunofluorescent techniques can be used. In these tests, antibodies specific for the neu gene product are contacted with the tissue sample (cells) under conditions appropriate for binding of the antibody to the neu gene product. Preferred antibodies are specific for the extracellular domain of the neu gene encoded product. The level of binding to the cell (which can be determined by standard immunohistochemical or immunofluorescent techniques), is indicative of the level of expression of the neu gene product.

The neu gene product can be detected in biological fluids by immunoassay techniques. These include competitive assays and immunometric (radioimmunometric or enzymetric) assays. A sample of biological fluid (i.e., blood, saliva) is obtained from the subject to be tested. The sample is contacted with antibodies specific for the neu gene encoded product under conditions which permit complexation of the antibody and the neu gene encoded product in the sample. The level of complex formation (antibody/antigen reaction) is indicative of the amount of neu gene encoded product in the sample. The amount of antibody-antigen complex can be measured by standard techniques. For example, in an immunometric assay a second labeling antibody is used to quantify the amount of antibody/antigen complex.

Immunometric assays by which human neu antigen has been detected in serum and plasma, as well as in normal, preneoplastic and neoplastic cells, are described in Example 7A to 7C. The "capture" immunoassays were carried out as described in detail in the Examples and, in general, a capture immunoassay for detection of human neu antigen in a human fluid carried out according to the present method includes:

a. forming an incubation mixture of a solid phase with a first monoclonal or polyclonal anti-neu antibody;

b. incubating the mixture under conditions appropriate for the anti-neu antibody to bind to the solid phase;

c. separating the solid phase from the fluid sample and eliminating (e.g., "blocking") any sites on the solid phase that do not contain anti-neu antibody;

d. forming an incubation mixture of the immunoadsorbent containing anti-neu antibodies with the human fluid containing neu antigen;

e. incubating the mixture under conditions and for a period of time sufficient for the human neu antigen in the fluid to bind to the immunoadsorbent;

e. separating the immunoadsorbent from the liquid sample;

f. incubating the immunoadsorbent with a second, labeled anti-neu monoclonal antibody; and

g. detecting the amount of label bound to the immunoadsorbent, using known techniques.

In particular, neu antigen can be detected using a forward capture immunoassay as detailed in Example 7. Briefly, polystyrene plates were coated with an anti-neu monoclonal antibody, or a combination of anti-neu monoclonal antibodies, the purpose of which was to capture the neu antigen from biological specimens. Once coated, sites not bound to the capture antibodies were blocked with buffered bovine serum albumin. Samples containing, or suspected of containing, neu antigen were incubated and a second anti-neu antibody labeled with biotin was added. To detect the biotin, a streptavidin horseradish-peroxidase solution was added along with a substrate for the enzyme and the optical density of the product was measured. The quantity of neu antigen was determined by comparing the optical density of the samples with a predetermined relationship between the quantity of neu antigen standards and the optical density of these standards.

As a result of these assay methods, a tornary complex is formed which includes: 1) a first anti-neu antibody, 2) the neu gene product ("neu antigen") and 3) a second anti-neu antibody. This second antibody is labeled either before or after formation of complex. The label can be attached directly or indirectly to antibody using any standard method. For example, the antibody can be complexed with biotin. In immunoassays of this invention capable of detecting the neu antigen, the first antibody can be an anti-neu monoclonal antibody, a mixture of anti-neu monoclonal antibodies, or a polyclonal anti-neu antibody.

The complex can be formed before it is immobilized onto a solid phase. In other embodiments, the complex can be immobilized on the solid phase at the same time that it is formed. In preferred assays, the neu gene product is immobilized on an immunoadsorbent which specifically "captures" or binds the gene product. This immunoadsorbent is formed by affixing to it an antibody specific for an idiotope of the neu gene product. Thus, for most purposes, two different anti-neu antibodies are used to form the immunoadsorbent and the labeled antibody.

Capture assays may be performed in forward, reverse or simultaneous mode. In a forward capture assay for a neu gene product, an anti-neu antibody is affixed to a solid phase. A liquid sample to be tested is incubated with the immunoadsorbent. Incubation is maintained for a sufficient period of time to allow the neu gene product in the liquid sample to bind to its immobilized anti-neu antibody on the immunoadsorbent. After this first incubation, the solid phase immunoadsorbent is separated from the sample. The immunoadsorbent is washed to remove unbound antibody and interfering substances, such as non-specific binding proteins, which may also be present in the liquid sample. The immunoadsorbent containing neu antigen bound to immobilized antibody is subsequently incubated with a labeled anti-neu antibody to form a ternary complex of: immobilized antibody-neu antigen-labeled antibody. The incubation is carried out for a period of time and under conditions sufficient to ensure binding of the labeled anti-neu antibody to the neu antigen. After the second incubation, another wash may be performed to remove unbound label from the solid phase immunoadsorbent. The labeled anti-neu antibody bound to the solid phase immunoadsorbent is then measured, and the amount of label detected serves as a direct measure of the amount of neu antigen present in the liquid sample.

The sandwich immunoassays may also be performed in reverse and simultaneous modes. In reverse modes, an incubation mixture is formed of the liquid sample to be tested containing neu antigen and a soluble labeled anti-neu antibody. The mixture is incubated, then contacted with a solid phase immunoadsorbent containing the same or different anti-neu antibody. After another incubation, the immunoadsorbent is separated from the mixture and the label bound to the immunoadsorbent is taken as an indication of the amount of neu antigen in the liquid sample.

In the simultaneous mode, an incubation mixture is formed of the liquid sample containing neu antigen to be measured, labeled anti-neu antibody and the solid phase immunoadsorbent. After appropriate incubation to form a ternary complex, the solid phase immunoadsorbent is separated from the mixture and the label associated with the immunoadsorbent is measured to given an indication of the amount of neu antigen in the liquid sample.

Tumor Therapy

The antibodies specific for the neu gene encoded product can be used to treat tumors. For therapy, the antibodies can be used alone in passive immunotherapy or as components of immunotoxins. In passive immunotherapy, an anti-tumor amount of monoclonal antibody is administered in a physiologically acceptable vehicle (e.g., normal saline) to a patient afflicted with a tumor which expresses the neu gene product (see Example 8). For example, antibodies specific for the extracellular domain of the neu gene product can be used for tumor therapy. For immunotoxin therapy, the antibody can be linked to an anti-cancer pharmaceutical or a cytotoxin to form an immunotoxin. Various pharmaceutical or cytotoxic agents can be covalently or noncovalently coupled to the antibodies. Examples of useful therapeutic agents include: radioactive compounds (e.g., isotopes of Boron and Rhenium); agents which bind DNA, such as alkylating agents or various antibodies (e.g., daunomycin, adriamycin, chlorambucil); anti-metabolites (e.g., methotrexate); and inhibitors of protein synthesis (e.g., diptheria toxin and toxic plant proteins).

The invention is further illustrated by the following Examples.

8

### Example 1: Isolation of neu cDNA clones and neu gene products

#### A. The neu gene family

Exposure of perinatal BDIX rats to a single dose of the alkylating agent ethylnitrosourea leads to a high incidence of neuroectodermal tumors. Rajewsky, M.F. et al., In:Origins of Human Cancer, Cold Spring Harbor Laboratory, 709-726 (1977); Rajewsky, M.F., In:Recent Results in Cancer Research 84:63-76 (1983). Up to 95% of animals mutagenized transplacentally after the fifteenth day or gestation or injected directly with ethylnitrosourea up to ten days after birth will develop central and peripheral nervous system tumors after a dose- and strain-dependent latency time. These tumors and the cell lines derived from these tumors display the characteristics of a wide variety of neural and glial cell types. Schubert, D., Nature, 249:224-227 (1974).

DNA isolated from four independently derived tumor cell lines of this type contains activated oncogenes which can be detected in an NIH 3T3 focus forming assay. The majority of oncogenes detected in this assay have been shown to be genetically altered versions of one of the three closely related ras genes (Varmus, 1984). However, the gene transferred from these neuro/gliobastomas is unrelated to the ras genes and has been designated neu.

Neu was first recognized to be a distinct gene by its association with the 185,000 dalton tumor antigen, p185, which is displayed on the surface of transfected cells. Neu is related in DNA sequence to the erbB gene, which encodes the epidermal growth factor (EGF) receptor, and antisera raised against the EGF receptor show some cross-reactivity with p185. However, detailed analysis has shown that neu bears only limited homology to erbB and that the two genes reside on different chromosomes. Schechter, A.L. et al., Science, 229:976-978 (1985). Thus, the neu gene is related to, but distinct from, the gene which encodes the EGF receptor.

cDNA clones of the neu oncogene have been isolated from cell lines transformed by this gene. Bargmann, C.I. et al., Nature, 319:226-230 (1986). Human versions of the same gene have also been isolated and termed variously c-erbB2 or HER2. The DNA sequences of these rat and human clones predict a 1260 amino acid protein product of the neu gene which is colinear with and 50% identical to the predicted amino acid sequence of the EGF receptor. By analogy to the EGF receptor, the neu product appears to be a transmembrane protein consisting or a cysteine-rich extracellular region of 650 amino acids, a transmembrane domain, and an intracellular portion of 580 amino acids consisting in part of a tyrosine kinase domain.

Biochemical studies of the p185 protein support these conclusions. p185 is glycosylated and accessible to antisera in intact cells, which is consistent with its being localized at the cell surface. It also has an associated tyrosine specific protein kinase activity. p185 does not, however, bind EGF and thus appears to be the receptor for an as yet unidentified growth factor.

#### B. Isolation of clones of normal and trans forming alleles of the rat neu gene

Biologically active genomic clones of normal and transforming alleles of the rat neu gene have recently been isolated. Hung, M.-C, et al., Proceedings of the National Academy of Sciences, U.S.A., 83:261-264 (1986). Structural comparison of these clones revealed no evidence of gross rearrangements, suggesting that subtle genetic alterations were responsible for activation of the neu oncogene. Comparable levels of p185 were shown to be expressed in nontransformed cell lines containing the normal allele and in transformed cell lines containing the mutant allele, suggesting that the alteration responsible for the activation of neu did not lead to deregulation of expression of the gene. Such results implicate a transforming lesion within the encoded protein p185, which should be represented in cDNA versions of the gene.

#### C. Comparison of cDNA clones of the normal neu gene and transforming cDNA clones

To determine the effect of the alteration responsible for activation of the neu gene, a comparison was made of the previously isolated transforming cDNA clone, DNAs from three other ethylnitrosourea-induced activations of the neu gene and a cDNA clone of the normal allele of neu.

#### Isolation of a normal neu cDNA clone

It was first necessary to isolate a normal neu cDNA clone. To do so, a cDNA library was constructed using RNA from the cell line DHFR G8. Hung, M.-C., et al., Proceedings of the National Academy of Sciences, U.S.A., 83:261-264 (1986). The DHFR cell line was made by transfecting a genomic cosmid clone containing a complete normal neu gene from the BDIX strain of rat into NIH 3T3 cells. These cells express high levels of the neu gene product, p185, and a high level of the neu RNA transcribed from the transfected gene. cDNA clones were made by the S1 snapback technique, tailed with dCTP using terminal transferase, and inserted into dG-tailed pBR322 at the PstI site. Thirty recombinant plasmids reactive with neu probes were isolated.

These plasmid clones were compared by restriction mapping to a full length cDNA clone of an activated neu oncogene. While these normal cDNA clones have common sequences with the previously identified transforming cDNA clones, none contained the entire coding region of neu. A clone containing the entirety of the neu coding region was constructed, however, by in vitro recombination of two partial, overlapping clones which share a unique NaeI site. The 5' end of the resulting clone was sequenced to verify the presence of the initiation codon for the neu-encoded p185 protein.

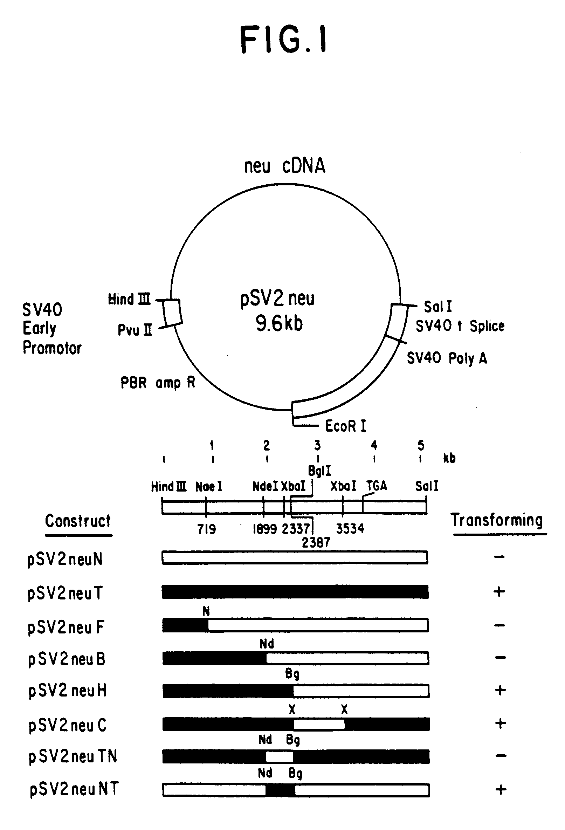

This normal neu clone was inserted into the pSV2 expression vector to create pSV2neuN, as shown in Figure 1. Mulligan, R.C. et al., Nature, 277: 108-114 (1979). A transforming neu cDNA clone derived from the B104-1-1 cell line, which is a secondary transfectant of an activated rat neu gene, was inserted into pSV2 to create a plasmid designated as pSV2neuT (Figure 1). pSV2neuT was highly active in a focus-forming assay on NIH 3T3 cells or Rat 1 fibroblasts. This assay measures the ability of DNA molecules that have been introduced into cells by the transfection to convert such cells, growing in monolayer culture to a transformed state, causing descendants of these cells to form a cluster or focus of morphologically transformed cells in an area of the cell monolayer. However, when the normal neu cDNA, also inserted into the pSV2 vector, was transfected into NIH 3T3 cells using identical conditions, no foci were observed.

Comparison of p185 production by transformed and nontransformed cells

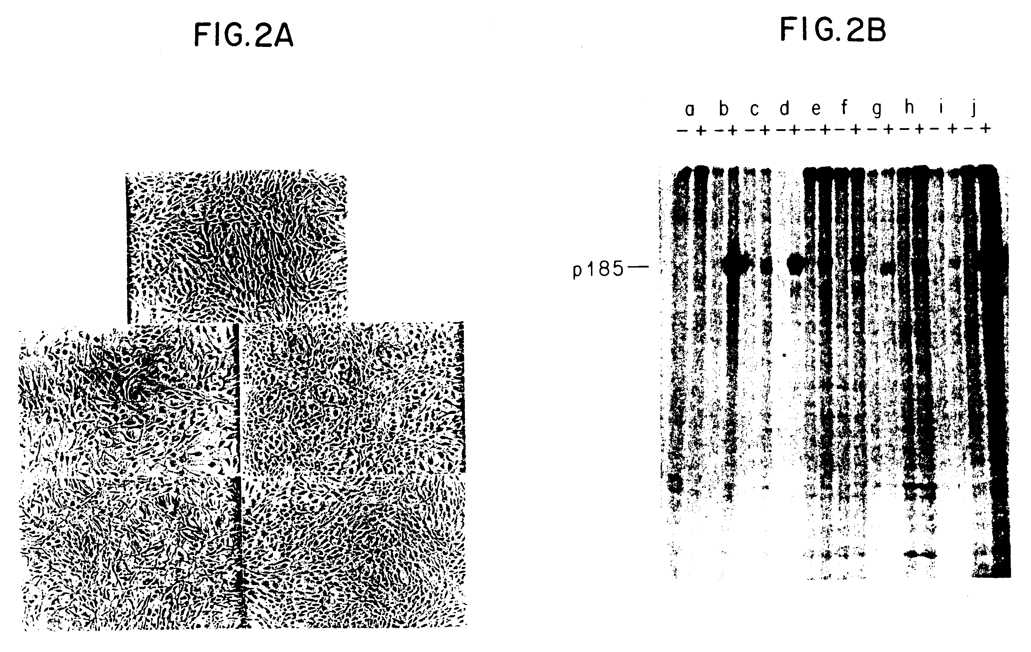

Cell lines containing the pSV2neuN or pSV2neuT plasmids were isolated by cotransfection with pSV2neo (referred to hereafter as the neo-r marker) and selection of G418. Southern, P.J. and P. Berg, Journal of Molecular and Applied Genetics, 1:327-341 (1982). Cell lines expressing the pSV2neuT construct were morphologically transformed and refractile; those containing pSV2neuN were flat and nontransformed in morphology. These cell lines were metabolically labeled with $^{32}$P orthophosphate and their lysates incubated with a monoclonal antibody that specifically precipitates the rat neu gene product. As shown in Figure 2B, lanes c, d, and i, the levels of labeled p185 were comparable in the transformed and nontransformed cells. These results are consistent with earlier work suggesting that neu has been activated by a mutation in the coding region of the gene rather than one which deregulates expression. Hung, M.-C. et al., Proceedings of the National Academy of Sciences, U.S.A., 83:261-264 (1986).

Example 2: Genetic analysis of the neu gene

A. Identification of the DNA sequences responsible for neu gene activation

Identification of sequences responsible for the transforming activity of the pSV2neuT clone was carried out by using recombinants between this clone and the pSV2neuN clone carrying the normal allele. The recombinants were constructed by ligation of appropriate cloned DNA segments. Figure 1 shows the structure of a series of clones which delineate the region of neu that carries the activating mutation. The struture of each recombinant clone shown was verified by restriction mapping. In each case, at least two independent plasmid isolates were tested for the ability to morphologically transform NIH 3T3 cells. All recombinant clones were cotransfected with the neo-r marker and the morphology of the resulting G418-resistant colonies was scored. Morphologically nontransformed colonies were tested to ensure that they were expressing structurally intact p185 protein from the acquired cDNA clones.

Clones pSV2neuF and pSV2neuB, which contain the first 719 and 1899 nucleotides, respectively, of the transforming clone fused to the remaining sequences of the normal clone (Figure 1), were not transforming, although they did direct the synthesis of p185. Clone pSV2neuH, which contained transforming neu sequences from the 5' end of the gene up to nucleotide 2387 and normal neu sequences thereafter, gave foci upon transfection and yielded transformed colonies indistinguishable from those generated by the parental pSV2neuT clone. pSV2neuC was also transforming. It contained an XbaI fragment from nucleotide 2337 to nucleotide 3534 of the normal neu gene, which replaced the corresponding portion of the transforming cDNA. These results indicate that the normal cDNA and the transforming cDNA differ in a sequence between nucleotides 1899 and 2337 and that the presence of this sequence in the transforming clone is necessary for transformation.

In order to prove that this sequences is also sufficient for transformation, the reciprocal constructs pSV2neuTN and pSV2neuNT (Figure 1) were constructed and tested by transfection into NIH 3T3 cells and Rat 1 fibroblasts. pSV2neuTN contained the entire transforming cDNA with the exception of nucleotides 1899 to 2387, defined by NdeI and BglII sites, which are derived from the normal neu clone. pSV2neuNT contained the entire normal neu cDNA sequence except for the corresponding 488 nucleotides, which were replaced by those from the transforming clone. In parallel experiments, pSV2neuNT gave comparable numbers of foci to pSV2neuT, the parental transforming clone; pSV2neuTN, pSV2neuN, and mock transfected controls yielded no foci. These experiments demonstrate that the essential genetic differences between the normal and transforming clones reside within this 488 nucleotide fragment.

The data shown in Figure 2 support these conclusions. G418-resistant cell lines isolated by cotransfection of the pSV2neu plasmids and the selectable neo-r gene are shown in Figure 2A. Lines containing pSV2neuTN or pSV2neuN are morphologically flat and indistinguishable from lines transfected with the neo-r gene alone. In contrast, cells containing pSV2neuNT and pSV2neuT are highly refactile and very similar to one another in morphology. These cell lines were metabolically labeled with $^{32}$P-orthophosphate and the resulting lysates incubated with anti-p185 monoclonal antibody 16.4. Drebin, J.A. et al., Nature, 312:545-548 (1984). Figure 2B shows the levels of p185 expressed in representative cell lines containing pSV2neuN (lanes c and d), pSV2neuT (lane i), pSV2neuTN (lanes e and f), and pSV2neuNT (lanes g and h). Lysates from DHFR G8 and B104-1-1 cells (lanes b and j), the cells lines from which the normal and transforming cDNAs were isolated, were also analyzed. Although individual cell clones show a wide range of p185 levels, it is clear that there are similar ranges of p185 expression in both the normal and transformed cells. No p185 is found in lines transfected with the neo-r marker clone alone (lane a). Thus, the differences in these cells must be accounted for by intrinsic differences in the properties of the p185 proteins that they express.

B. Definition of the mutation distinguishing the normal neu allele and the activated neu gene

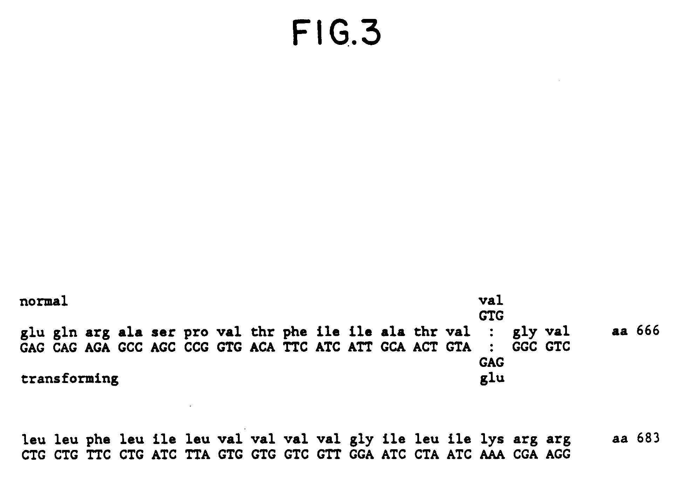

The complete DNA sequence of the coding region of the transforming neu cDNA has been determined. Bargmann, C.I. et al., Nature, 319:226-230 (1986). To define the precise mutation that distinguishes the two alleles, the DNA sequence of the region between nucleotides 1899 and 2387 was determined for the normal neu cDNA. Only a single difference was found between this sequence and that previously determined for the transforming clone. At nucleotide position 1012 there is an A in the oncogene clone, while the normal clone carries a T in this position. As a result, the predicted amino acid present at residue 664 of the encoded p185 is affected; a valine found in the normal protein is replaced by a glutamic acid in the oncogenic version. Figure 3 shows the DNA and predicted amino acid sequence of nucleotides 1968 to 2073 for both the normal neu gene and the transforming neu gene. The presumed mutation falls within the putative transmembrane domain of the neu gene product, p185.

Genomic clones of normal and transformining alleles of neu have been previously isolated. These clones were used to independently verify the nucleotide difference seen in the cDNAs. Such corroborative data served to exclude the possibility that the observed difference in the cDNAs arose during cDNA cloning. Sequencing of subclones of the genomic versions of the two alleles confirmed that the same T to A substitution was present in these genomic clones. This indicates that the mutation, a T to A transversion, arose somatically during creation of the B104 neuroblastoma tumor or cell line.

Example 3: Activation of neu oncogene

A. Determination of activation of independent neu oncogenes

Earlier results had shown that DNAs prepared from four out of six neuro/gliobastoma cell lines displayed activated neu oncogenes in a NIH 3T3 focus assay. Shih, C. et al.: Nature, 290:261-264 (1981). These six cell lines had been derived by transplacental mutagenesis of BDIX rat embryos with ethylnitrosourea. These independent activated neu genes were evaluated to see if they contain the same activating mutation. DNAs from transfectants containing these neu genes were hybridized with nucleic acid probes which would recognize preferentially one or the other allele of the neu gene. This technique has been successful in identifying various activated alleles of ras genes. Bos, J.L. et al., Nature, 315:726-730 (1985); Zarbl, J. et al., Nature, 315:382-385 (1985).

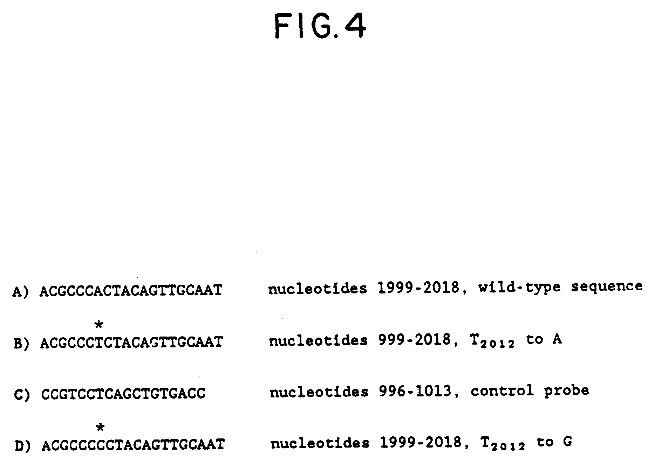

Nucleic acids corresponding in sequence either to the wild type or the mutant neu version of the neu gene were synthesized. The sequence of these two 20-mers is given in Figure 4. These 20-mers were then hybridized under stringent conditions (2 degrees below the calculated $T_m$ of a perfect duplex) to dried agarose gels containing DNAs which had been digested with appropriate restriction endonucleases. The 20-mer corresponding to the wild type sequences hybridized approximately ten times as well to pSV2neuN as it did to pSV2neuT. In contrast, the nucleic acid whose sequence derived from the transforming allele preferentially hybridized to pSV2neuT by the same factor.

DNA was isolated from transfectants carrying the four independently activated neu oncogenes described above and cleaved with HindIII. The resulting fragments were resolved on a 1% agarose gel. The agarose gels were incubated under conditions identical to those used in analysis of the cloned DNAs described above shown in Figure 4. DNA from DHFR G8, which contains about 50 copies per genome of the normal genomic neu gene, was included as a control.

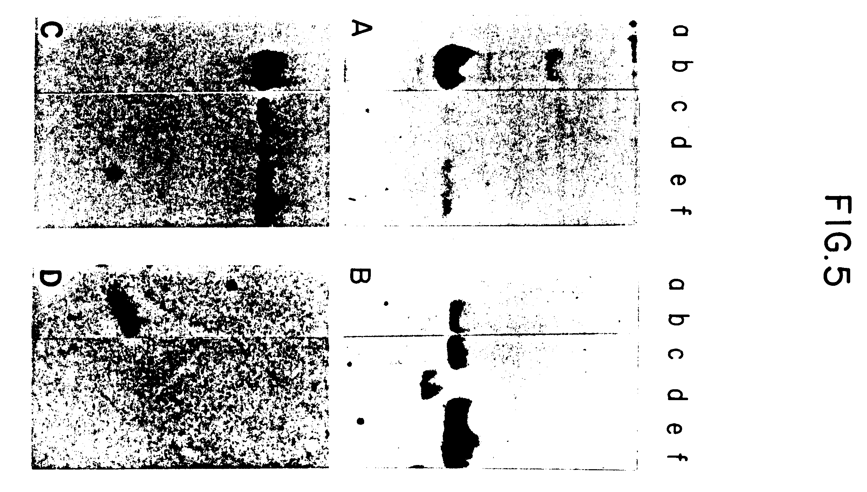

Figure 5A shows hybridization of the transfectant DNAs with the nucleic acid corresponding to the wild type sequence. A strong signal appears in DHFR G8 DNA, which has introduced copies of the normal neu gene (lane b). Considerably weaker signals can be seen in lanes containing the neu-transformed transfectant DNAs (lanes c-f) and in untransfected NIH 3T3 cells (lane a). Figure 5B shows an identical gel prepared in parallel probed with the nucleic acid containing the mutant sequence. DHFR G8 DNA reacts only weakly with this probe (lane b) but all neu-transformed transfectant DNAs (lanes c to f) yield strong signals with this probe. The transfectant DNA shown in lane d has a smaller neu-homologous HindIII fragment than the other transfectants due to truncation of the transfected gene at the 3' end.

A low level of reactivity between the wild type probe and the transfectants is probably due to cross-reactivity of the probe, since it is comparable to the signal seen upon analysis of cloned pSV2neuT DNA with the same probe (compare Figure 4 lanes b and d with lanes c-f in Figure 5A and 5B).

In order to control for differences in signal intensity due to DNA loading and transfectant copy number, the gel in 5B was stripped of probe and rehybridized with a nucleic acid probe from a different part of the neu gene. The transfectant cell lines may contain different copy numbers of the rat neu gene due to variable amplification during the process of transfection. The results of this hybridization are shown in Figure 5C. Comparison of corresponding lanes in Figures 5A, B, and C shows that DNAs of all neu oncogene transfectants exhibit stronger hybridization with the mutant probe than with the wild type probe, and that the extent of their hybridization with the mutant probe correlates well with the copy number of the neu gene present in the various DNAs.

Although these data strongly suggest that all of the activated neu genes studied have alterations at the same nucleotide position, it was not clear whether the mutations involved the same T to A transversion in all cases. A T to G transversion might give the same pattern of hybridization, since it would cause an A/G mismatch with the wild type nucleic acid and a relatively stable G/T mismatch with the mutant nucleic acid. To address this possibility, a third 20-mer was synthesized with the sequence shown in Figure 4. This nucleic acid was incubated under hybridizing conditions with the gel analyzed in Figure 5A after the initial probe had been removed. This nucleic acid did not hybridize preferentially to the transfectant DNAs under stringent conditions (Figure 5D). Thus, the alteration in each of these activated neu genes is probably the same T to A transversion.

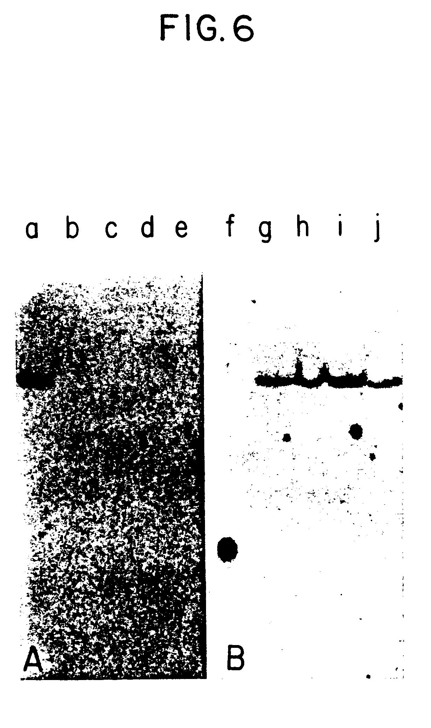

That the point mutation was generated during tumorigenesis and thus did not reflect preexisting polymorphism in the rat genome or activation during the transfection process was also confirmed. DNA was isolated from BDIX rat liver and from the four rat tumor cell lines which were derived from BDIX rats and yielded activated neu genes in a transfection assay. Duplicate gels were probed either with the wild type or the mutant nucleic acid (Figure 6). BDIX liver DNA reacts well with the wild type but not with the mutant nucleic acid (Figure 6, lanes a and f). In contrast, the four tumor cell lines react only with the mutant nucleic acid (Figure 6, lanes b-e and i-l). This demonstrates that the T to A transversion at nucleotide 2012 arose in the generation of the tumor or tumor cell lines. It also seems that the tumor cell lines are hemizygous or homozygous for the activated allele of the neu gene. Loss of the normal allele may have occurred during tumorigenesis or in passage of the tumor cell lines. A similar loss or under-expression of the normal ras alleles has been observed in several tumors and tumor cell lines that contain oncogenic versions of these ras genes. Capon, et al. Nature, 304:507-513 (1983): Guerrero, I et al., Proceedings of the National Academy of Sciences, U.S.A., 82:7810-7814 (1985).

Multiple independent activations of neu

Chemical carcinogenesis, though provoked by apparently randomly acting agents, often results in specific genetic changes in the resulting tumor cells. Neu appears to be activated by the same nucleotide change in each of the four of the transforming neu alleles analyzed. Each of these alleles was isolated from a neuro- or glioblastoma that arose from transplacental exposure to the alkylating agent ethylnitrosourea. Schubert, D. et al., Nature, 249:224-227 (1974). Neu is also activated at the same residue in four independent nervous system tumors induced by the related alkylating agent methylnitrosourea.

Similarly, extensive study of methylnitrosoureainduced mammary carcinogenesis in Buf/N rats has shown that among nearly 100 independent resulting tumors, all contain H-ras oncogenes activated by the identical G to A transition at residue 35. Sukumar, S. et al., Nature, 306:658-661 (1983); Zarbl, H. et al., Nature, 315:382-385 (1985). Other examples of specificity of activation include dimethylbenzanthracene-induced papillomas with activated H-ras genes in genetically susceptible Sencar mice and thymic lymphomas induced by gamma irradiation or methylnitrosourea, which yield transfectable K-ras and N-ras oncogenes, respectively. Balmain, A. and I.A. Pragnell, Nature, 303:72-74 (1983); Guerrero, I. et al., Proceedings of the National Academy of Sciences, U.S.A., 81:202-205 (1984). The K-ras oncogenes detected in the former case appear to have the same activating mutation in at least three out of four tumors examined. It is interesting to note that the administration of the same mutagen, methylnitrosourea, under different conditions leads to the specific activation of H-ras in mammary tumors, N-ras in thymic lymphomas, and neu in neurectodermal tumors. Guerrero, I. et al., Science, 225:1159-1162 (1984).

The carcinogen which induces each of these tumors must initially inflict widespread damage to the cellular DNA. The final mutation detected is, however, highly specific. Clearly, strong biological forces must act during multistep carcinogenesis to select the outgrowth of cells bearing the genetic lesions observed in the ensuing tumors. It appears that only a small proportion of cellular genes can be converted into biologically active oncogenes. Within one of these genes, only a few of many possible mutations will yield an actively transforming oncogene. The relatively rare mutations which do generate oncogenes are enriched first because they must confer a selective advantage to the tumor, and later because they can be detected in the focus-forming assay.

The repeated appearance of a specific lesion in one tumor type suggests the presence of additional forces selecting among the possible activating mutations. The nature of the activating mutation must be strongly influenced by the chemical reactivities of the carcinogen. For example, different mutagens used to induce thymomas lead to different activated genes in the resulting tumors. Similarly, mammary carcinomas induced by dimethylbenzanthracene contain a different specific alteration from those induced by methylnitrosourea.

The reactivity of ethylnitrosourea, the agent which induced the described neuroblastomas, allows it to form a number of different adducts in DNA. Singer, B. and J.T. Kusmierek, Annual Review of Biochemistry, 52:655-693 (1982). Among these, an adduct causing G to A transition mutations is well described. Rajewsky, M.F., Recent Results in Cancer Research, 84:63-76 (1983). Such mutations have been found repeatedly as the activating lesions in the H-ras oncogenes of mammary carcinomas induced by the related carcinogen methylnitrosourea. Zarbl, H. et al., Nature, 315:382-385 (1985). The presently described mutations are, however, T to A transversions, the creation of which must be explained by alternative mechanisms involving another one of the many adducts formed after administration of these alkylating agents. This type of mutation is not without precedent: two other ethylnitrosourea-induced mutations isolated after germ-line mutagenesis of mice has also been shown to be T to A transversions. Popp, R.A. et al., Genetics, 105: 157-167 (1983); Lewis, S.E. et al., Proceedings of the National Academy of Sciences, U.S.A., 82:5829-5831 (1985). An N-ras oncogene isolated from a methylnitrosourea-induced lymphoma has been found to be activated by a C to A transversion suggesting that several different adducts induced by alkylating agents can lead to mutations. Guerrero, I. et al., Proceedings of the National Academy of Sciences, U.S.A., 82:7810-7814 (1985).

Example 4: Detection of neu oncogenes using nucleic acid probes

A. Examination of tumor DNAs from Buffalo rats with nucleic acid probes homologous to the neu gene

Sukumar and Barbacid (National Cancer Institute, Bethesda) have isolated ten methylnitrosourea-induced nervous system tumors of Buffalo rats which contain activated neu genes. Transfectants which arose from these tumor DNAs were assessed to determine whether the mutation in these neu genes was identical to that found in the tumors previously described.

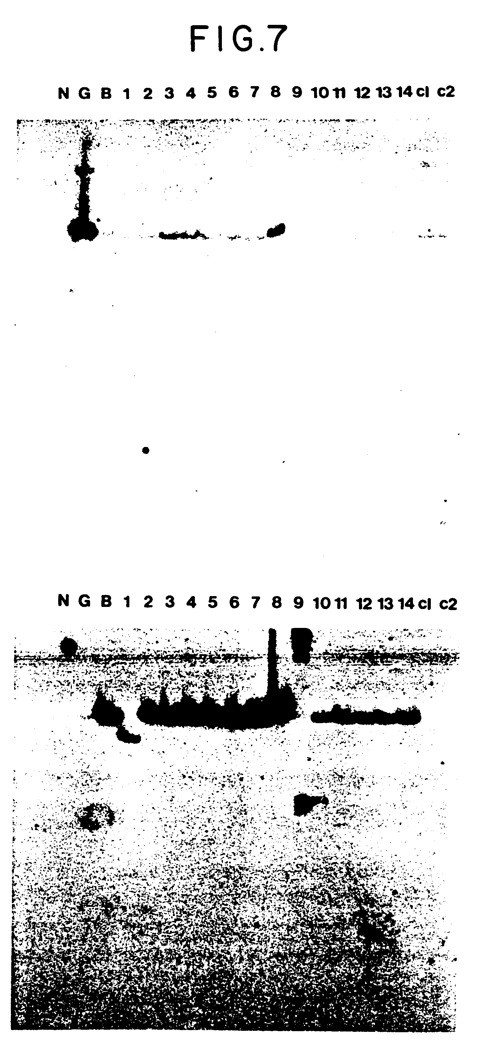

This was carried out in the following manner; Fourteen transfectants containing ten independent activated neu genes are shown in Figure 7 (tracks 1-14). Duplicate gels were probed with the nucleic acid probes described above. That is, they were probed either with the normal nucleic acid (Figure 7, top) or the nucleic acid corresponding to the transforming gene (Figure 7, bottom). NIH DNA (track N), DHFR G8 DNA (track G) (high copy of the normal neu gene0, B104-1-1 DNA (track B) (high copy of the transforming neu gene) and two Buffalo rat tumors which do not contain active neu genes (tracks c1 and c2) were included as controls. All of the tranfected neu genes bore the identical alteration as that in B104-1-1 DNA, the cell line from which the transforming neu cDNA clone described above was derived. Based on their differential hybridization to the nucleic acid corresponding to the transforming gene, the transfected neu genes are presumed to have the same sequence change as the B104-1-1 neu gene. These results provide ten independent examples of activated neu genes detected with the nucleic acid; demonstrate that a second mutagen, methylnitrosourea, leads to the activation of neu genes at this position in a similar way as ethylnitrosourea; and demonstrate that activated neu genes arise in Buffalo rats as well as BDIX rats.

B. Oligonucleotide-directed mutagenesis

Oligonucleotide-directed mutagenesis was used to substitute a glutamine residue for the valine residue normally found at amino acid position 664. When this mutated neu gene was inserted into an expression vector and transfected into or infected into recipient cells, 100% of the stable cell lines generated were transformed. (In contrast, 0.1% of recipient cells transformed with the normal neu gene (proto-oncogene), which encodes a protein including a valine residue at amino acid position 664, were transformed.) Thus, like glutamic acid, glutamine at position 664 leads to generation of a transforming neu gene, and mutations detected with a nucleic acid probe that would recognize glutamine would be presumed to be transforming mutations. Substitution of an aspartic acid residue at position 664, followed by transfection or infection into nontransformed cells, led to the transformed phenotype in 2-3% of the recipient cells. This appears to represent a weakly transforming allele of the neu gene, whose overexpression must be coupled to the mutation for transformation to occur.

C. Identifying the activation mutation in human neu genes

Although the discussion to this point, as well as the experimental work described, has been related to the point mutation responsible for activation of the rat neu oncogene, it is possible to use the same approach in identifying the corresponding activation mutation in human neu genes and in detecting the presence of a neu oncogene (or its corresponding proto-oncogene) in human tumor cell DNAs. For example, nucleic acid probes can be constructed which are specifically reactive with the region of a human neu gene which corresponds to the region shown to contain the point mutation responsible for the activation of the rat neu oncogene. An example of a probe which can be constructed is one based on a single nucleotide difference between a neu oncogene and its proto-oncogene, this single nucleotide alteration being responsible for conversion of the neu proto-oncogene into its activated oncogene form. These probes, which can be of any length, but will generally be 15 to 20 nucleotides long, can then be used to analyze a tumor cell genome, to determine whether it carries lesions (mutations) in the neu oncogene and to determine their precise location, as described above for the rat neu oncogenes.

D. Detecting the presence of a neu oncogene

An assay for detecting carcinogenesis caused by mutation of a neu proto-oncogene into neu oncogene comprises employing a labelled nucleic acid probe specific for a nucleotide sequence present in (or transcribed from) the proto-oncogene or the oncogene, but not the other. An assay for carcinogenesis in human cells can be performed by isolating DNA from the test cells and contacting the DNA with a labelled polynucleotide probe specific for either an oncogenic or protooncogenic sequence in the DNA and thereafter determining whether the probe hybridizes to the DNA. After being radiolabelled, these probes can be used, for example, in the Southern blot procedure to assess tumor cell DNAs for the occurrence of such point mutations. This type of assay can be used in a clinical context as a diagnostic tool to determine the profile of oncogenes activated in human tumor DNAs. The assay would be highly specific because it is capable of detecting single nucleotide alterations in genes of the neu family, thus providing very definitive information about the tumor cells being assayed.

Because of the change in amino acid sequence of the product protein encoded by a proto-oncogene from the product encoded by an oncogene, it is possible to detect either by specific serological reagents. The serological reagents can be specific for the normal, neu proto-oncogene-specified amino acid sequence at this site of the protein, or be specific for the altered oncogene-specified amino acid sequence at this site of the protein. Other serological reagents could be employed that are reacted with a region of the protein that is unaltered, and consequently reactive with either normal or abnormal forms of the encoded protein.

Using cloning techniques, significant amounts of the protein encoded for by the normal site of the proto-oncogene, or by the altered site of the oncogene, can be isolated. Such protein segments could be used to produce antibodies by standard antibody production techniques. Thus, for producing polyclonal antibodies, such proteins would be employed to immunize a host, such as a rabbit or a rat, and antibodies to the protein would be collected from serum obtained from the host.

Alternatively, monoclonal antibodies could be produced employing cells which produce antibodies to the protein produced by the isolated gene segment in typical fusion techniques for forming hybridoma cells. Basically, these techniques involve the fusing of the antibody-producing cell with a cell having immortality, such as a myeloma cell, to provide a fused cell hybrid which has immortality and is capable of producing the desired antibody (in this case, an antibody to the normal or altered segment of protein coded for by the isolated gene segment). The hybrid cells are then cultured under conditions conducive to the production of antibody after which antibody is collected from the cell culture medium. Such techniques for producing monoclonal antibodies have been well described in the literature. See, for example, U.S. Patent Nos. 4,172,124 and 4,196,265, issued to Hilary Koprowski et al., the teachings of which are hereby incorporated by reference.

Example 5: Cloning of Human neu gene

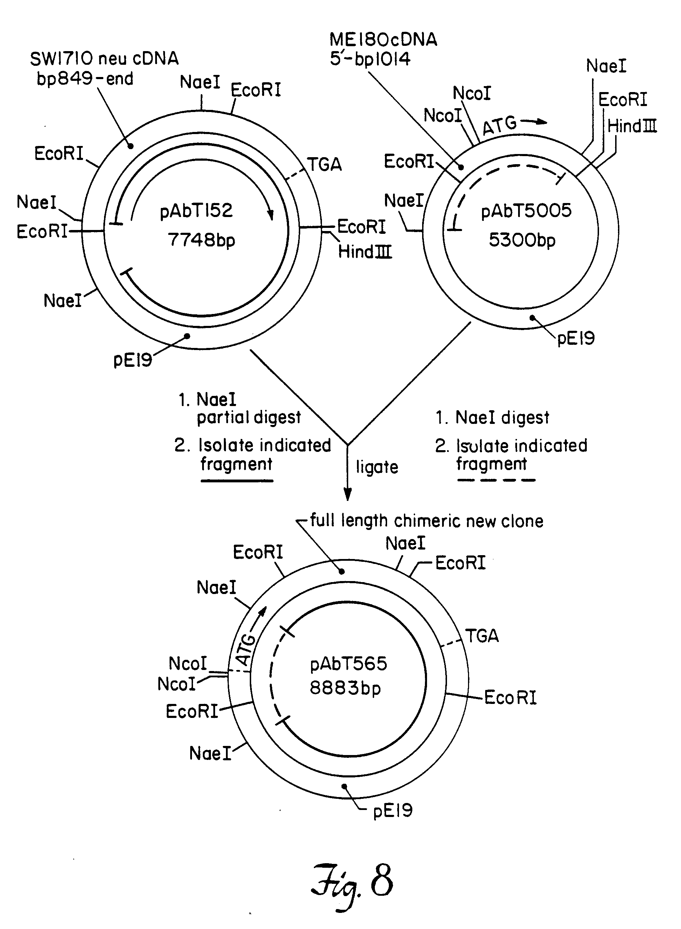

The human neu gene was cloned from two lambda gt11 cDNA libraries derived from poly A+ RNA of two cervical carcinoma cell lines (SW1710, ME180) both of which express high levels of neu RNA. A 1300 bp Pst 1 fragment of the rat neu tyrosine kinase domain was used as the probe to screen the SW1710 cDNA library. Two recombinant plaques were identified as human neu by restriction analysis and partial sequencing. The longer of the two clones contained the majority of the neu coding sequence. The recombinant sequence starts at nucleotide 849 and codes for all but the first 225 of the 1255 amino acids of the neu protein. The missing region of the human neu gene was isolated from the ME180 cDNA library using the 5' Eco R1 fragment of the SW1710 neu cDNA clone as a probe. The subcloned regions of the two clones and the scheme for the construction of a full length human neu gene are shown in Figure 8.

A. Construction of neu exoression vectors

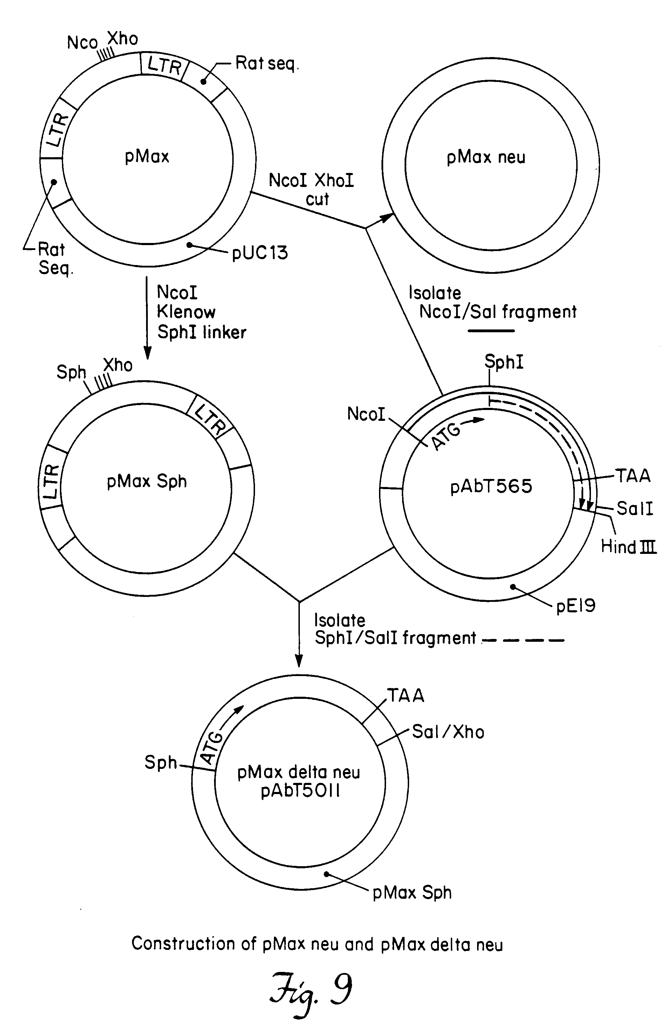

The entire neu coding sequence was cloned into the expression vectors pLJ (Roberts, et al., 1985, J. Virol., 56:404-413) and pMax (obtained from Bernard Mathey-Prevot without reservations, shown in Figure 9). Both these vectors put the neu gene under the transcriptional control of the molony murine leukemia virus promoter and enhancer (LTR). The pLJ vector also expresses $neo^R$ from an SV40 promoter.

B. Construction of the Transmembrane point mutation

Oligo-directed mutagenesis was carried out using the Amersham site-directed mutagenesis kit as per the manufacturers directions and using the following oligonucleotide:

CTGCGGTGGAGGGCATTCTG

the underlined nucleotides are different from the normal human neu allele. The presence of the point mutation was determined by differential hybridization and confirmed by sequence analysis using the Promega double stranded sequencing kit as per the manufacturers directions.

C. Construction of the truncated neu allele

The pMax vector was restricted with NCO 1, at a unique site in the polylinker, and filled in to create blunt ends with klenow, as shown in Figure 9. A 12 base pair Sph-I linker (N.E.B. #1115) with the following sequence: CATGCATGCATG was inserted at this site to generate a unique Sph-I site in the polylinker. This particular linker was chosen because it provides a translation initiation signal (ATG) in all three reading frames. The resulting vector is shown as pMax-Sph in Figure 9. PMax-Sph was restricted at the Sph-I site and at the Xho-I site (both in the polylinker). A Sph-I/Sal-I 2225 base pair fragment containing the neu cDNA from just 5' of the transmembrane region to the 3' end of the gene was isolated from a plasmid (shown as pAbT 565 in Figure 9) containing the entire human neu cDNA. The restricted vector and the isolated fragment were ligated together and transformed into the bacterial strain HB101. The resulting plasmid, shown in Figure 9, has been designated pMax delta neu or pAbT 5011.

D. Transfection of cells

Neu expression vectors were transfected into NIH 3T3 cells by the standard calcium phosphate procedure. Briefly, on day 1 cells are plated at $5 \times 10^5$ per 10 cm tissue culture dish. On day 2 the cells are refed and 4 hours later the DNA precipitate is added and the cells are incubated for 6 hrs and refed. The following day the cells are split into G418 containing media and incubated for 10-14 days. NIH 3T3 DNA was used as carrier and pSV-2 neo was used as the selective marker.

### E. Isolation of RNA

Total cellular RNA was isolated by the guanidinium isothiocyanate/CsCl method. When isolating RNA from cells in culture the lysis buffer (4M guanidimium isothiocyanate, 50 mM Tris-HC1 pH 7.5, 10 mM EDTA, 0.5% Sarkosyl, 0.1M BME) was added directly to the tissue culture dishes. When frozen tissue was the starting material the tissue was homogenized directly in the lysis buffer with a tissue homogenizer (Biospec products, INC.). The cellular lysate is then passed through a 18 gauge needle and layered on a 1.2 ml cushion of 5.7 M CsC1 in 0.1 M EDTA in a SW50.1 Beckman pollyallomar tube. Centrifuge at 35,000 rpm for at least 12 hours. The RNA pellet is then resuspended in TE containing 1% SDS.

### F. Hybridization probe

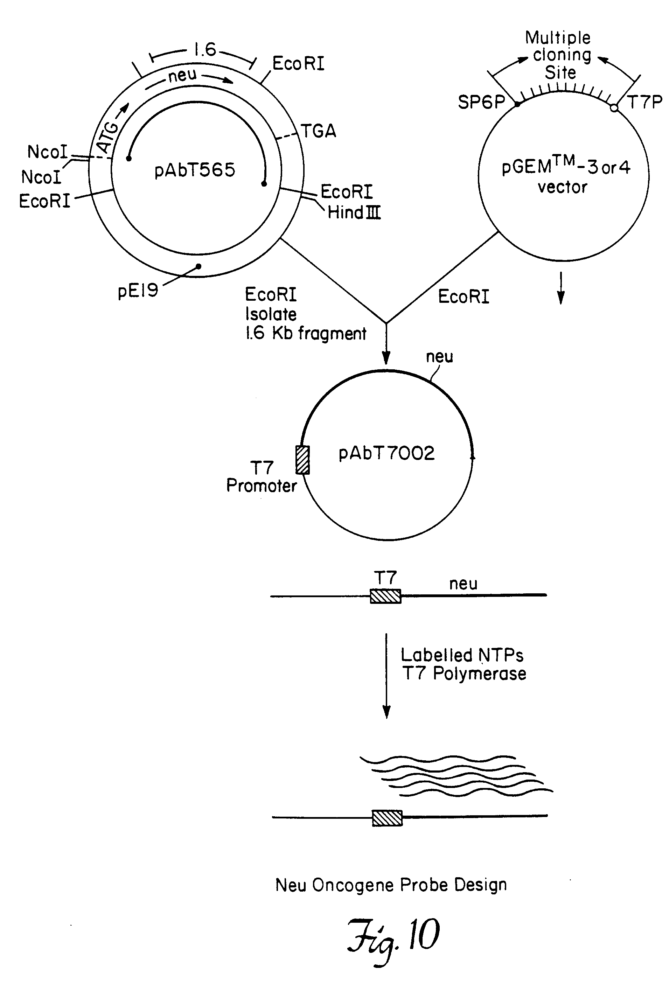

The 1.6 kb Eco RI fragment containing a portion of the extracellular domain, the transmembrane domain and a small portion of the tyrosine kinase domain was subcloned into Promega's pGEM dual transcription vectors as shown in Figure 10. Labelled RNA transcripts to be used as hybridization probes were generated according to the manufacturers directions.

### G. RNA slot blot analysis

RNA samples were diluted to 1 ug/ml in 6 X SSC, 7.4% formaldehyde and heated at 65°C for 15 min., then placed on ice. The slot blots were done using the S$S slot blot apparatus and the precut BA 85 nitrocellulose sheets supplied according to the manufacturers directions. The filter was prewet with water and then 10 X SSC and the RNA was added as a series of 2 fold dilutions starting at 200ng/slot in a volume of 200ul. The slots were then washed with 200 ul 10 X SSC and the filter was baked for 2 hours at 80°C in a vacuum oven. The filter was then wet with 10 X SSC and prehybridized for 2 hours in hybridization buffer (50% formamide, 5 X SSC, 50 mM Sodium Phosphate, 250 ug/ml; salmon sperm DNA, 5 X Denhardts). The hybridization solution was then replaced with fresh solution containing 1-2 X $10^6$ cpm/ml denatured $^{32}$P labelled RNA probe specific to human neu. The filters were then incubated overnight at 65°C. The following morning the filters were washed 3X in 0.1 X SSC, 0.1% SDS for 20 minutes at 80°C.

### H. Cloning of human neu alleles and testing transforming activity

Two cDNA libraries made from cervical carcinoma cell line RNA that express high levels of neu RNA were screened with a probe derived from the tyrosine kinase domain of the rat neu cDNA. Comparison of restriction maps and partial sequence data with published data confirmed the identity of the intact human neu clone constructed from the original isolates from this screen. The human neu cDNA was then transferred into an LTR driven expression vector. Cells transfected with this human neu construct expressed a 185,000 dalton protein that was indistinguishable from the authentic human neu expressed by the human mammary carcinoma cell line, SKBR-3. When this normal human neu clone (pMax neu shown in Figure 10) was transfected into NIH 3T3 cells they became transformed. This is in direct contrast with the data described for the rat neu gene. In the rat, no matter what the expression level, the normal rat neu allele was not transforming. Expression of the normal human neu gene in other expression vectors which expressed neu at lower levels did not lead to transformation of the NIH 3T3 cells. However, if the human neu gene was mutated so the homologous amino acid to the valine in rat neu was changed to glutamic acid, this gene transformed cells in either expression vector. This indicates, that the mutation activates the transforming activity of the human neu gene. Expression of the truncated human neu allele in either expression vector also transforms NIH 3T3 cells. These results are identical to those reported by Di Fiori, et al. Science, 1987; 237:178-182. In summary, both normal and mutated human neu alleles can transform NIH 3T3 cells although much higher expression levels are required for the normal neu.

### I. Analysis of neu RNA levels in Human cell lines and mammary tumors

Total cellular RNA was isolated from a variety of human cell lines and mammary tumors by the guanidium isothiocyanate method. The RNA was then subject to slot blot analysis using a human neu specific probe, the 1.6 kb Eco R1 fragment which contains a portion of the extracellular domain, the transmembrane domain and part of the tyrosine kinase domain. All RNA samples were also hybridized with a alpha tubulin probe to normalize for the amount of RNA present on the filters. The human mammary cell line HBL 100 (ATCC HTB 124) was used as a standard and all results are relative levels as compared to HBL 100. E. Gaffney, Cell Tissue Res., 229:563-568 (1982). The neu expression levels varied from between 1 to 64 times the HBL 100 levels. These results are shown in Table 1.

## Table 1

### Expression Levels of Neu-Specific RNA in Various Cell Lines

| Human Cell Lines | Neu Levels |
|---|---|
| HBL-100 | 1X |
| ME180 | 8X |
| SW1710 | 4X |
| MKN-7 | 32X |
| SK-BR-3 | 64X |
| A-431 | 1-2X |
| BT-483 | 8X |

### Transfected Mouse Line

| | |
|---|---|
| 18-3-7 | ++++ |
| NIH3T3 | 1X |

HBL-100 is an epithelial cell line derived from the milk of a nursing mother.

ME180 is a cervical carcinoma cell line.

SW1710 is a cervical carcinoma cell line.

MKN-7 is a gastric cancer cell line.

SK-BR-3 is an adenocarcinoma of the breast.

A-431 is an epidermoid carcinoma cell line.

BT-483 is a ductal carcinoma of the breast.

18-3-7 are NIH3T3 cells transfected with neu.

The results were similar to those previously published (Kraus, et al., <u>EMBO</u>, 1987; <u>6</u>:605-610). We have also analyzed the <u>neu</u> RNA levels in a series of primary human mammary carcinomas. Again we find a wide range of <u>neu</u> expression levels ranging from less than 1 to 128 times the standard, as shown Table 2.

Table 2

| Quantitation of <u>Neu</u>-Specific RNA levels from Mammary Tumor Samples | | |
|---|---|---|
| | Sample | Neu RNA Level |
| 1. | 6-8-6-86 | 128 |
| 2. | 8-11-20-86 | 64 |
| 3. | 6-10-15-86 | 16 |
| 4. | 5-11-13-86 | 16 |
| 5. | 9-9-10-86 | 16 |
| 6. | 12-9-12-86 | 8 |
| 7. | 5-10-10-86 | 8 |
| 8. | 8-8-7-86 | 8 |
| 9. | 1-7-7-87 | 8 |
| 10. | 5-9-3-86 | 4 |
| 11. | 4-2-4-87 | 4 |
| 12. | 6-6-12-87 | 2 |
| 13. | 12-12-17-86 | 2 |
| 14. | 13-9-15-86 | 2 |
| 15. | 21-2-27-87 | 2 |
| 16. | 4-12-5-86 | 1/2 |
| 17. | 11-12-15-86 | 1/8 |
| 18. | 10-1-23-87 | 1/8 |

Hybridization results were determined by serial dilutions of total cellular RNA in dot blot hybridization analysis using the $^{32}$P <u>neu</u> probe. Numbers have been normalized for total RNA amount by hybridization with tubulin probe. Levels are relative to the normal mammary cell line HBL-100 which is set at 1X.

As above, the levels are expressed as relative levels compared to HBL 100 and normalized for tubulin expression.

Example 6: Production of neu-specific monoclonal antibodies

Production of Hybridomas

The hybridomas described below were generated by immunization of mice with viable cells (the 18-3-7 cell line described below) which express the full length protein encoded by the <u>neu</u> oncogene. This is an important distinction from other approaches for the generation of monoclonal antibodies. Using the full length protein presented by viable cells as the immunogen, it is possible to generate a collection of monoclonal antibodies with specificities that span the entire length of the extracytoplasmic domain of the protein. This is as opposed to the use of peptide immunogens, or short polypeptides generated by prokaryotic systems, which present only a limited number of epitopes from the original protein, and hence raise an immune response of limited specificities. Furthermore, by presenting the protein antigen in its native state, the immune system will be responding to an antigen which most closely resembles that which will be seen when the antibodies are later used for diagnostic or therapeutic applications.

B. Generation of 18-3-7 Cells

18-3-7 cells are a transfected NIH 3T3 cell line that express full length normal human <u>neu</u> at levels equal to or greater than the human mammary carcinoma cell line, SKBR-3. The human <u>neu</u> gene is expressed by a Murine leukemia virus LTR (promoter and enhancer). This cell line exhibits all the characteristics of transformed NIH 3T3 cells. They grow in soft agar, form tumors in nude mice and display altered morphological characteristics. This cell line was used as the immunogen for the isolation of anti-<u>neu</u> specific monoclonal antibodies.

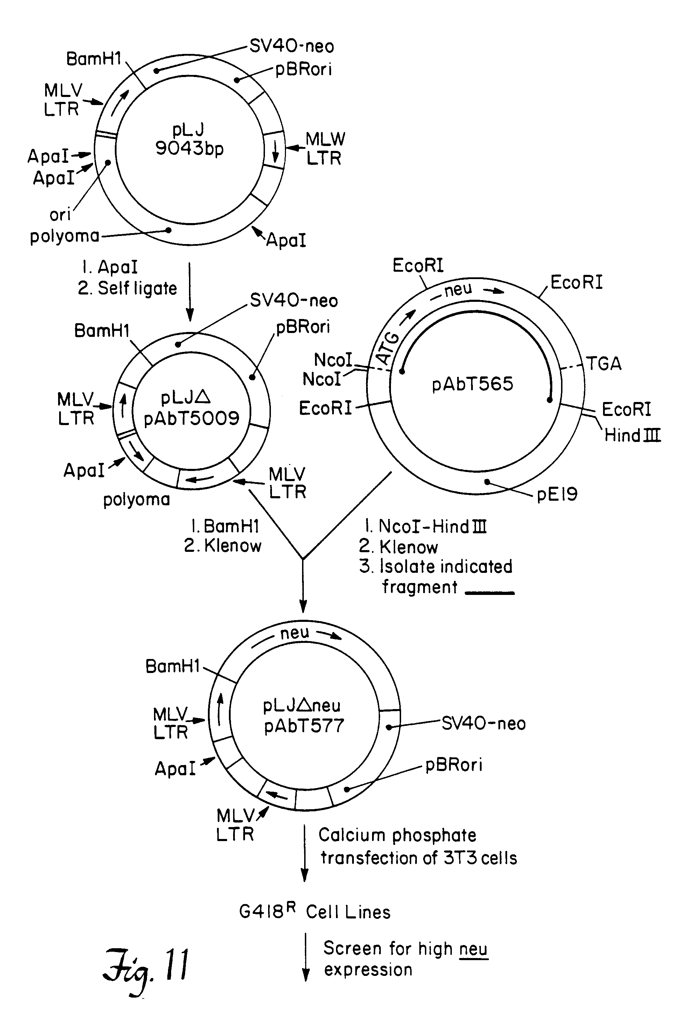

The pLJ retroviral vector was modified to remove the polyoma early region, thereby eliminating the endogenous transforming activity of the pLJ vector. Construction of the modified vector is shown in Figure 11. The modification was accomplished by restricting pLJ with Apa I and isolating the 6300 base pair fragment, and recircularizing it with $T_4$ ligase. The resulting plasmid (pdelta LJ or AbT 5009, shown in Figure 11) was digested at the unique Bam HI site, filled in with Klenow, and ligated to a NcoI-HindIII filled in fragment containing the entire human neu protein coding region. The resulting plasmid (pdelta LJ neu or pAbT 577, shown in Figure 11) was transfected into NIH 3T3 cells by the calcium phosphate precipitation procedure. Transfected cells were selected in G418 (pdelta LJ has a SV40 promoted neo$^R$ gene). The colonies were screened for neu expression by RNA dot blots. 18-3-7 was one of highest expressors out of approximately 50 screened.

### C. Immunization of Mice

Two adult female Balb/c mice were immunized intraperitoneally (I.P.) with 1.4 X $10^6$ viable NIH3T3 cells per animal. This was followed immediately by an I.P. injection of cyclophosphamide in $H_2O$, 30 mg/kg. The cyclophosphamide treatment was repeated 24 and 48 hours after the primary injection. On day 14 following immunization, the mice were injected I.P. with 1.5 X $10^6$ viable 18-3-7 cells. The animals were allowed to rest for another 14 days, at which time the entire sequence of injecting NIH3T3 cells, cyclophosphamide, and 18-3-7 cells was repeated. Four days following the second injection of 18-3-7 cells, the animals were sacrificed and their spleens obtained for the first fusion. A second, identical experiment was performed, in four female Balb/c mice and four female CB6 (Balb/c X C57BL/6) mice, using 1.8 X $10^6$ NIH3T3 cells, and 4.8 X $10^6$ 18-3-7 cells per mouse in the first round, and 8.5 X $10^6$ NIH3T3 cells and 2.7 X $10^6$ 18-3-7 cells in the second round of immunizations.

### D. Hybridoma Methodology

Hybridomas were produced by fusing cells from immunized mice with SP2/O myeloma cells (ATCC CRL 1518) by a polyethylene glycol (PEG) method. Spleens were removed aseptically from immunized mice, and a single cell suspension of the spleen cells was obtained by perfusing the spleen with serum-free media (DME). Spleen cells and SP2/O cells (harvested from a log phase growth culture) were mixed together at a ratio of 5:1, spleen cell:myeloma cell. The cells were centrifuged at 200 X g for 10 minutes at 4°C, and the supernatant removed by aspiration. After loosening the cell pellet by gently tapping the bottom of the tube, 1 ml of sterile, 37°C, 10% PEG in DME was added dropwise. The tube was gently swirled while adding the PEG over a 1.5 minute period. An additional 10 ml of 37°C serum-free DME was then added dropwise, followed by another 20 ml of media. The suspension was then centrifuged at 200 X g for 10 minutes at room temperature. Media was aspirated from the cell pellet, and media containing peritoneal macrophages (2 X $10^4$ cells per ml) in the presence of 20% fetal calf serum, 0.2 mM hypoxanthine, 0.4 uM aminopterin, and 0.032 mM thymidine (HAT media) was used to resuspend the cell pellet. (Peritoneal macrophages were obtained from unimmunized mice, either Balb/c or CB6, depending on which spleen cells were used for fusion. These cells were obtained by injecting and immediately removing serum-free media into the peritoneum of euthanized animals.) The post-fusion cells were resuspended to a final cell concentration (not including the peritoneal macrophages) of 5 X $10^5$ cells/ml. One milliliter of this cell mixture was distributed to each well of 24 well plates.

### E. ELISA Procedure and Preliminary Screening

Hybridomas which grew after the fusion procedure were initially screened for the secretion of anti-neu antibodies by an ELISA assay on a cell lysate of 18-3-7 cells. Lysates were prepared by incubating freshly harvested 18-3-7 cells in the presence of a hypotonic lysis buffer (10 mM Tris, 10 mM KC1, 5 mM EDTA, pH 8.0) followed by the addition of Triton X 100 to a final concentration of one percent. A lysate of NIH3T3 cells was prepared similarly for use as a negative control. Microtiter plates (Nunc, Immunoplate II) were coated overnight at room temperature with 50 ul of lysate, at a total protein concentration of 500 ug/ml. After aspirating to remove unbound antigen, ELISA's were performed by first incubating 50 ul of culture supernatant obtained from the viable hybridoma colonies in the antigen-coated microtiter wells. A 3 hour incubation at 37°C was followed by 3 washes with a washing buffer (0.5% Tween 20, 20 mM Tris, pH 7.6) and then a one hour incubation at 37°C with 50 ul horseradish peroxidase labelled goat anti-mouse IgG + IgA + IgM (HRP-GAM-GAM). The wells were again washed three times with washing buffer, and the assay was developed by the addition of 50 ul of a tetramethylbenzidine (TMB) solution. This solution was prepared by dissolving 10 mg of TMB in 1 ml of dimethlysulfoxide (DMSO), and adding 100 ul of this solution to 5 ml of TMB buffer (0.1 M sodium acetate, to pH 6.0 with 0.1 M citric acid) along with the addition of 10 ul of 3% hydrogen peroxide. Color was allowed to develop for 5 minutes, at which time the enzymatic reaction was stopped by adding 50 ul of 2 N $H_2SO_4$. The optical density (OD) of the resulting yellow color was read at 450 nm on a microtiter plate reader. A positive reaction, as indicated by a greater yellow color developed on 18-3-7 cell-coated wells than on NIH3T3 cell-coated wells, signaled that there was antibody present in the culture supernatant which recognized the neu oncogene product.

F. Subcloning Hybridomas

Hybridomas which yielded positive results upon initial screening were expanded and cloned by limiting dilution to assure that the cells and resulting antibodies were indeed monoclonal. A feeder cell population was first prepared by obtaining thymocytes from 6 week old unimmunized mice, and making a single cell suspension at a concentration of $2 \times 10^4$ cells/ml in HAT media. Hybridoma colonies which tested positive for the presence of antibody to the neu gene product were diluted in the media containing thymocytes to a concentration of 5 hybridoma cells/ml. Two hundred microliters of this solution was then delivered to each well of 96 well microtiter plates. Once colonies had grown, the supernatants were again tested for the presence of antibody to the neu oncogene product. If the results were positive when tested by the ELISA assay as described above, the colonies were cloned by limiting dilution a second time.

Hybridomas which were obtained in the manner described above following the first fusion secrete monoclonal antibodies which have been designated BD5-2d, TA1-1c, RC1-4c, NA3-6a, and OD3-10j. Following the second fusion, hybridomas were obtained which secrete antibodies named PB3, RC6-2, NB3, ID5, and IB3-4.

G. Antibody Isotype and Subclass Determination

ELISA assays were performed to determine the isotype and light chain class of the antibody produced by the hybridomas, and to determine the IgG subclass. For this purpose, a kit was purchased from Boehringer Mannheim (Indianapolis, IN) which contained all of the necessary immunoreagents. Tissue culture supernatants obtained from the cloned hybridoma colonies were incubated on lysates of 18-3-7 cells as described above. This was followed by an incubation with goat antisera specific for mouse immunoglobulin isotypes, light chain classes, and IgG subclasses, and then with horseradish peroxidase labelled swine anti-goat IgG as the second antibody. The assay was developed using ABTS (2,2'-azino-bis-[3-ethylbenzthiazoline-6-sulfonic acid]) as per the manufacturer's instructions, and the OD of the resulting green color was read at 405 nm.

Using this method, it was determined that 3 of the monoclonal antibodies from the first fusion, BD5-2d, RC1-4c, and TA1-1c, are $IgG_1$/kappa antibodies, and NA3-6a, and OD3-10j are IgM/kappa antibodies. The monoclonal antibodies RC6-2, NB3, ID5, and IB3-4 obtained from the second fusion are $IgG_1$/kappa and the antibody PB3 is $IgG_{2a}$/lambda.

H. Radioimmunoprecipitation

Immunoprecipitation of radioactively labelled 18-3-7 cells was done using each of the monoclonal antibodies to determine whether the antibodies recognized a protein of 185 kd molecular weight, the expected molecular weight of the neu oncogene product. A near confluent monolayer of 18-3-7 cells (or NIH 3T3) cells in a 10 cm petri dish was incubated overnight in media containing 500 uCi of $^{35}$S-labelled cysteine. The cells were harvested the following morning, and were lysed in a detergent buffer (IP buffer: 1% Triton X-100, 1% sodium deoxycholate, 0.1% SDS, 10 mM Tris, 0.65 M NaC1, pH 7.2) containing the protease inhibitors PMSF and soybean trypson inhibitor. Approximately 1 uCi of the labelled cell preparation was then incubated overnight at 4 C with 500 ul of culture supernatant from each of the hybridomas. During this incubation period, 50 ug of purified rabbit anti-mouse IgG (Kirkegaard & Perry Labs) was mixed with 50 ul of a 1:1 slurry of Protein A-Sepharose (Pharmacia) in IP buffer overnight at 4°C. The excess rabbit antibody was removed by washing the Protein A-Sepharose once with IP buffer, and the slurry was then added to the incubation mixture containing the labelled cells and the monoclonal antibody. This mixture was allowed to react overnight at 4°C. The Protein A-Sepharose was pelleted by centrifugation and was washed four times with IP buffer, followed by one wash with TBS (10 mM Tris, 150 mM NaC1, pH 8.2), and the pellet was allowed to dry. Each pellet was resuspended in 50 ul of sample buffer for SDS gels (10 mM Tris, 4% SDS, 20% glycerol, 10% 2-mercaptoethanol, 0.04% bromphenol blue). One half of each of the samples was run on SDS polyacrylamide gels, using a 4.5% acrylamide stacking gel, and a 7% separating gel. The gels were dried and then autoradiographed.

Results of the immunoprecipitations indicated that all of the monoclonal antibodies recognized a protein of approximately 185 kd molecular weight in the 18-3-7 cells which was not present in the NIH 3T3 cells. This was determined by the presence of a dark band on the autoradiograph which corresponded to the distance travelled in the gel by a 185 kd molecular weight protein as indicated by standard protein markers. A similar experiment was done using SKBR-3 cells (a human breast carcinoma) and A431 cells (a human epidermoid carcinoma). The SKBR-3 cells have been shown by other investigators to express high levels of the human neu oncogene product, and immunoprecipitations with the monoclonal antibodies described above yielded confirming results. The band observed migrated the same distance as the band which was precipitated from the labelled 18-3-7 cells. The A431 cell line, on the other hand, is known to express very high levels of the human epidermal growth factor receptor (EGFR), which is a 170 kd protein that has significant homology to the human neu oncogene product in the tyrosine kinase domain of the proteins. This is the one protein which might be cross-reactive with the neu gene product if the antibodies recognize the tyrosine kinase region. However, immunoprecipitation of A431 cells with the monoclonals described above showed no reactivity in the area of 185 kd or 170 kd. A control antibody, specific for human EGFR, did react with a protein in the A431 cells, as expected, and the band observed corresponded to 170 kd molecular weight.

Because there was no reactivity with A431 observed when the monoclonal antibodies raised to the 18-3-7 cells were used, it was concluded that the antibodies were specific for the human neu oncogene product, and did not cross react with the human epidermal growth factor receptor.

Example 7: Detection of p185 neu antigen in biological samples

These Examples illustrate detection of the human neu antigen (approximate molecular weight of 185,000 daltons and referred to as p185) in human fluids such as serum, plasma or urine or in normal preneoplastic or neoplastic cells.

A. Capture Immunoassay

Polystyrene plates (Costar, Cambridge, MA) were coated with either 1 microgram (ug) of an anti-neu monoclonal antibody (Mab), a combination of anti-neu Mabs, or a polyclonal anti-neu antibody for the purpose of capturing the neu antigen from various biological specimens. Mabs were diluted in carbonate buffer (pH = 9.6) and 100 microliters (ul) added to each well of the microtiter plate. The plates were then incubated overnight at 37°C.

After incubation the plates were washed three times with phosphate buffered saline (PBS) with 0.1% bovine serum albumin (BSA). Plates were then incubated with a solution of PBS with 2.5% BSA in order to block sites on the microtiter wells not coated with the anti-neu antibody. Plates were incubated for 1 hour at 37°C and again washed three times with PBS/0.1% BSA. If plates were not used they were stored at 4°C until use.

Specimens to be evaluated for the neu antigen consist of lysates prepared from normal, preneoplastic or neoplastic cells or bodily fluids such as serum, plasma or urine. Specimens were incubated with the anti-neu Mabs coated on the microtiter wells in order to capture the neu antigen from the specimen. Specimens were incubated overnight at 25°C. After the overnight incubation, plates were washed 6 times with PBS/0.1% BSA to remove the excess biological specimen.

Another anti-neu Mab labeled with biotin was added to each well for a 1 hour incubation at 37°C. Plates were washed 6 times with PBS/0.1% BSA. To detect the biotin labeled anti-neu Mab, a streptavidin horseradish-peroxidase solution at a 1:4000 dilution was added for 1 hour at 37°C. Plates were washed 6 times with PBS/0.1% BSA. To complete the reaction the substrate OPD was added for 15 minutes at 37°C. The reaction was stopped with sulfuric acid and the optical density was determined using a Nunc plate reader in a wavelength of 490 nm.

B. Detection of neu from cell and tumor lysates using capture assays

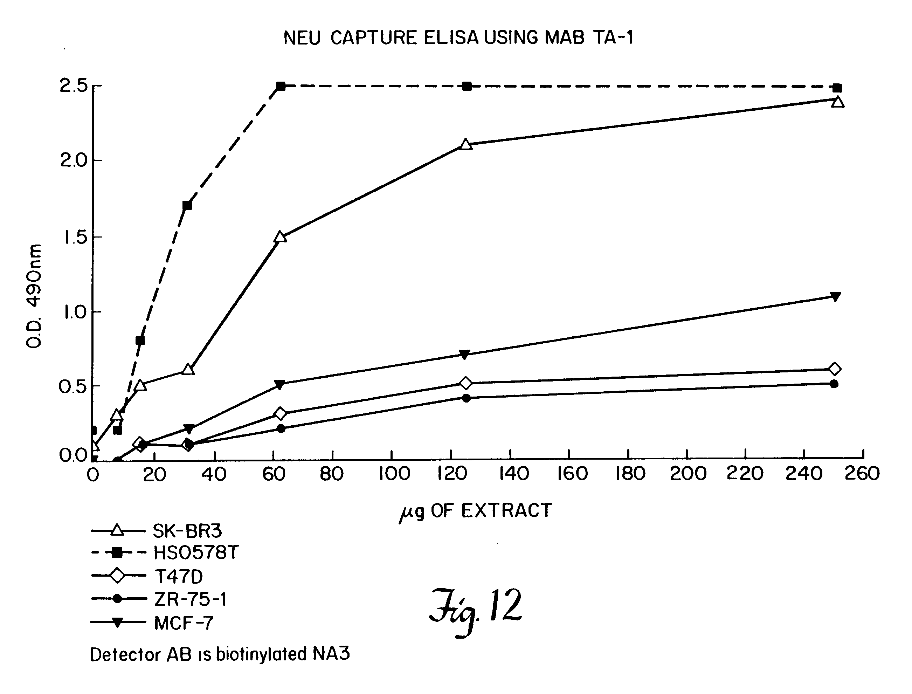

Several capture immunoassays have been performed to determine usefulness of this assay on biological materials. Figure 12 shows the results of a capture immunoassay in which the first antibody is TA-1 and the second antibody is biotinylated NA-3. Cell lysates were prepared from several human tumor cell lines. Neu RNA levels have been published for several of these cell lines (SK-BR-3, ZR-75-1, MCF-7).

EP 0 412 116 B1

The relative levels of neu antigen detected by this assay are in agreement with the published RNA data. The results of these assays and several others (not shown) using cell lines with known levels of neu antigen indicates this assay can be used to determine the relative level of neu antigen in cell lysates. Results also indicate that differences in expression of neu antigen can be used to classify the carcinoma cell lines shown in Figure 12.

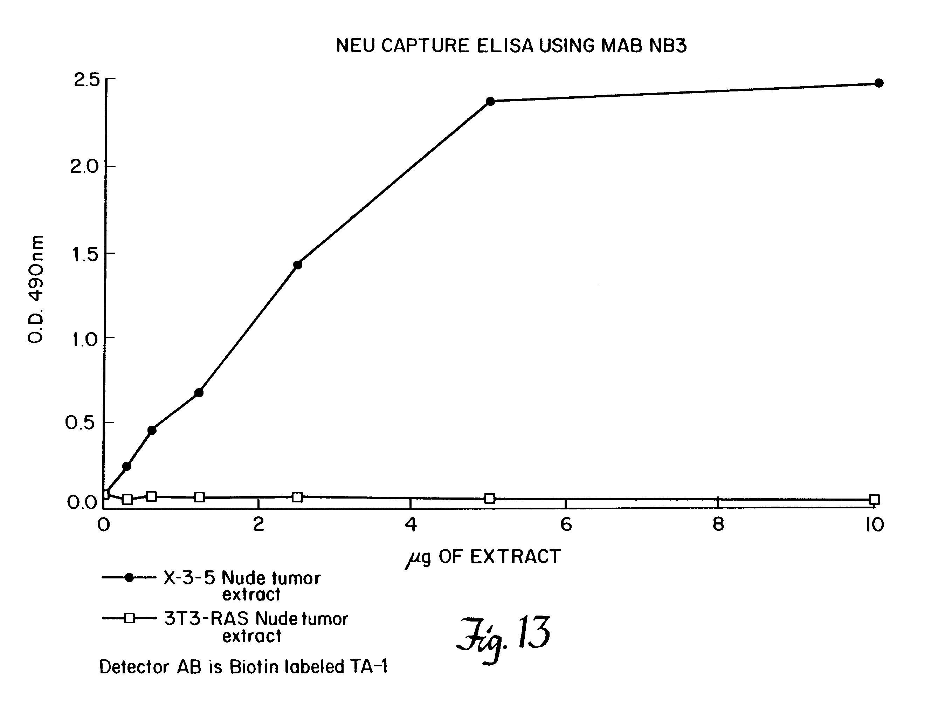

In order to determine if this assay could detect neu antigen in tumor lysates, tumors that either expressed neu (X-3-5) or did not express neu (3T3-ras) were grown in nude mice. The two NIH 3T3 derived cell lines are isogenic except that X-3-5 contains an expressed human neu gene. Figure 13 shows the results of a capture immunoassay using NB-3 as the first antibody and biotinylated TA-1 as the detector antibody. Neu antigen was detected in the lysate of the X-3-5 tumor but in the lystate of the 3T3-ras tumor, indicating the assay can specifically detect the human neu antigen in tumor lysates.

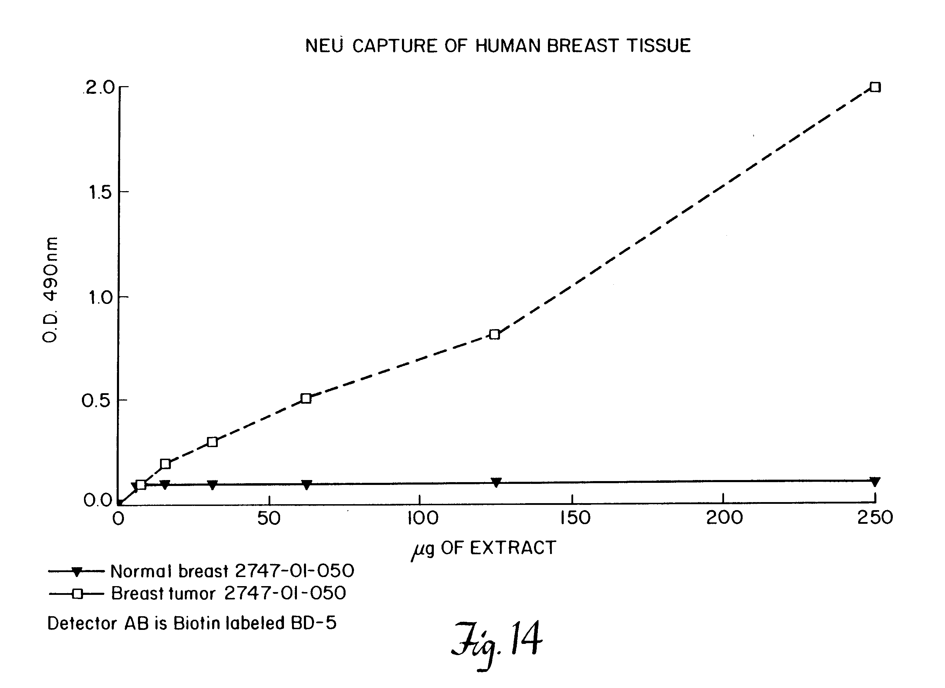

Several investigators have shown that many human breast tumors express neu at high levels. In order to determine if neu can be detected in human breast tumors two samples from the same individual were prepared. Lysates were prepared from a human breast tumor (2747-01-050) and from normal breast tissue (2747-01-050) from the same patient. In this assay TA-1 was used as the first antibody and biotinylated BD-5 was used as the detector antibody. Figure 14 shows that neu can be detected in the tumor but not the normal breast tissue.

These assays show that the neu capture immunoassay can specifically detect human neu from either cell or tumor lysates. The data also indicate the assay can determine relative levels of neu between samples.

C. Detection of neu in blood plasma and sera

These examples illustrate detection of neu antigen in sera and plasma from mice and humans bearing neoplastic tumors.

In order to determine if neu antigen can be specifically detected in human sera or plasma several control experiments were performed. These include detection of the neu antigen in the culture supernatant of cell lines that express high levels of neu and in the sera of nude mice bearing tumors.

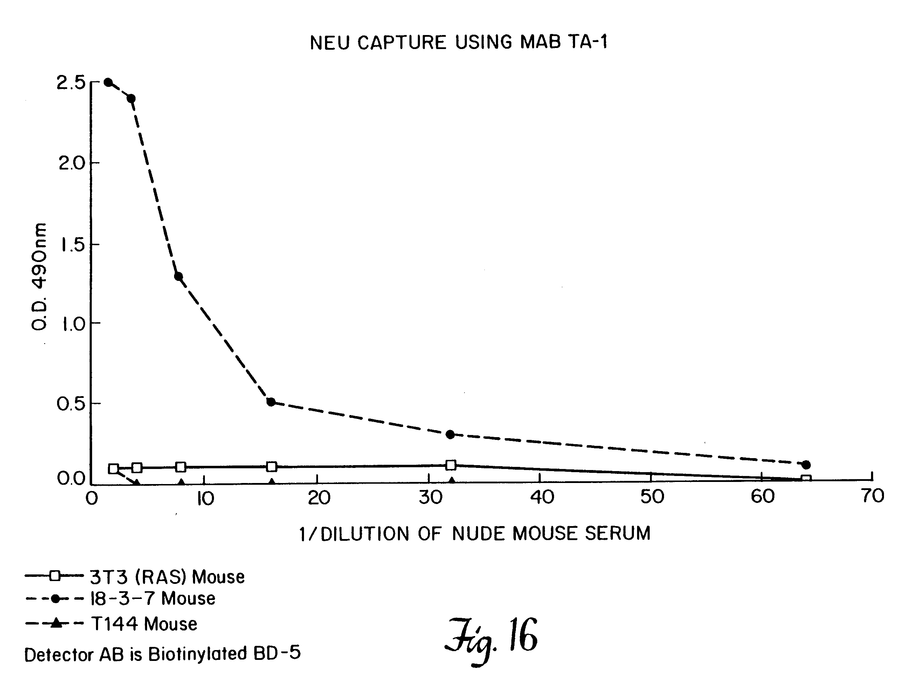

Figure 15 shows the results of a capture immunoassay of neu from culture supernatants of cell lines using NB-3 as the first antibody and biotinylated TA-1 as the detector antibody. The results show that neu antigen can be detected in the supernatant of murine (18-3-7) or human (SK-BR-3) cell lines that express high levels of neu but not in the supernatant of a cell line that does not express neu, 3T3-ras, or in media alone. Two of these cell lines are able to grow as tumors in nude mice (18-3-7 and 3T3-ras). Mice bearing tumors derived from injecting these cell lines subcutaneously into nude mice were bled and their sera was analyzed for the presence of neu antigen by a capture immunoassay using TA-1 as the first antibody and biotinylated BD-5 as the detector antibody. The results of this assay are shown in Figure 16. As with the cell or tumor lysates and the cell culture supernatants only the sera of the nude mouse bearing a tumor that expresses neu shows neu antigen in the assay. Both normal nude mouse sera and sera from a nude mouse bearing a tumor that does not express neu show no neu antigen in the sera.

These experiments indicate that neu antigen is found in the sera of nude mice bearing tumors expressing neu antigen. The neu antigen was found in the supernatant of human cell lines expressing neu - (SK-BR-3) as well as the cell line causing the nude mouse tumor.

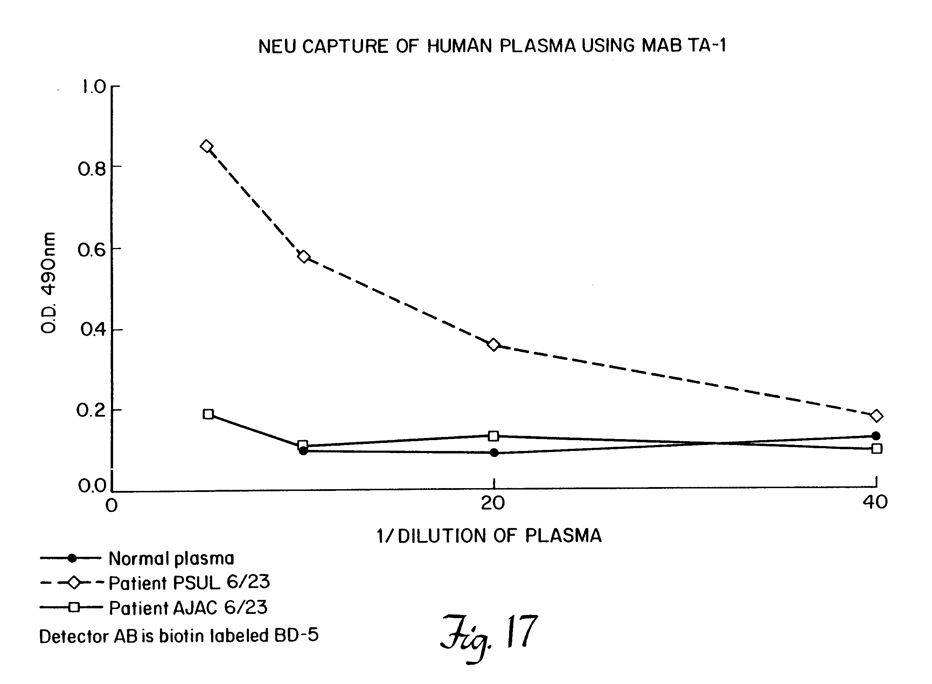

Assays were performed that were designed to test the hypothesis that patients with tumors expressing high levels of neu will have sera containing neu antigen. One series of assays used anti-neu Mab TA-1 as the capture reagent and BD-5 labeled with biotin as the detection reagent. Samples for analysis included normal human plasma and plasma from 2 breast carcinoma patients. Results show that normal plasma and plasma from patient AJAC were virtually unreactive in this assay whereas plasma from patient PSUL showed significant reactivity in this assay suggesting that the p185 antigen was present in the plasma of the breast carcinoma patient PSUL (Figure 17).

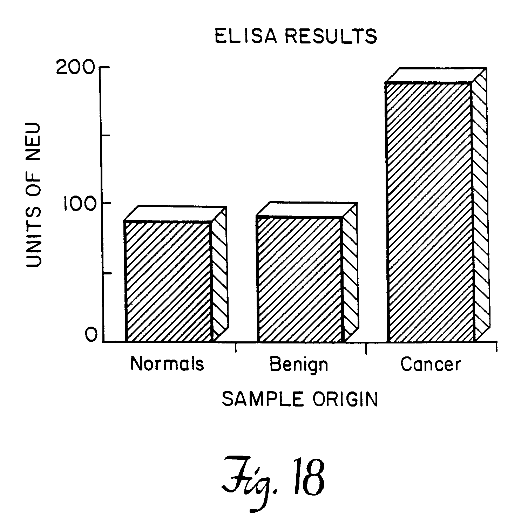

This experiment was repeated on a larger number of patients with MAb NB-3 as the capture reagent (affixed to the solid support) and MAb TA-1 labeled with biotin as the detection reagent. Using this assay, 37 separate plasma samples were evaluated for reactivity with neu-specific monoclonal antibodies. The specimens consisted of 12 plasma samples from normal people, 6 plasma samples from individuals with benign breast disease and 19 plasma samples from breast cancer patients. Figure 18 illustrates the average value (units of neu) for the three groups. Normals and benign have average values between between 100-110 and the 19 breast cancer patients had neu values that averaged approximately 200. Table 3 presents the individual data obtained for samples. Several neu values from patients with breast cancer exceeded the upper limit of the assay and are designated as "500 + " in Table 3. In these cases, samples were further

22

diluted by a factor of 2, and the assays were re-run. Results are shown in parentheses.

TABLE 3

| ELISA Results (neu units) from Three Types of Patients | | | |
|---|---|---|---|
| Patient | Normal | Benign | Cancer |
| 1 | 100 | 90 | 210 |
| 2 | 90 | 100 | 210 |
| 3 | 137 | 87 | 500 + (300) |
| 4 | 75 | 70 | 120 |
| 5 | 112 | 102 | 200 |
| 6 | 183 | 110 | 500 + (210) |
| 7 | 152 | | 175 |
| 8 | 77 | | 500 + |
| 9 | 91 | | 142 |
| 10 | 88 | | 70 |
| 11 | 81 | | 60 |
| 12 | 63 | | 80 |
| 13 | | | 90 |
| 14 | | | 90 |
| 15 | | | 122 |
| 16 | | | 142 |
| 17 | | | 160 |
| 18 | | | 123 |
| 19 | | | 140 |

Example 8 TA-1 Inhibition of neu Transformed Cells in Soft Agar

This Example illustrates that anti-neu monoclonal antibodies may have cytostatic effects on transformed cells.

Mouse fibroblasts (NIH3T3 cells) transfected with and expressing high levels of the human neu gene will form colonies in soft agar. This property is directly related to the amount of neu protein and its inherent tyrosine kinase activity. Monoclonal antibodies (MAbs) which recognize the extracellular portion of the human neu protein will cause these proteins to cluster and patch on the cell surface, and then internalize into the cell thereby decreasing the amount of tyrosine kinase activity. We have demonstrated that the addition of the MAb TA-1 to a neu transformed NIH3T3 cell line (17-3-1-3) growing in soft agar will decrease the number of colonies formed by this cell line in a concentration dependent manner. Specifically, at the highest concentration of TA-1 used (150 ug/ml), less than 5% of the expected number of colonies formed (7 colonies compared with the 150 formed on the untreated control group). A nonspecific MAb matched to the same mouse subtype as TA-1 (both IgG$_1$) had no effect on the number of colonies formed at 150 ug/ml. This growth inhibitory effect appears to be cytostatic and not cytocidal as the cells which do not form colonies in the soft agar are still viable. This result has obvious implications for antibody directed toxin therapy to tumor cells which over express the human neu protein.

Deposit

A deposit was made with the American Type Culture Collection, Rockville, Maryland of the antibody-producing cell line BD5-2d. The ATCC number is HB 9698.

**Claims**

1. An immunoassay for detecting the overexpression of human neu gene encoded p185 protein in a sample of cells comprising:
   (a) obtaining a lysate of the sample of cells;

23

(b) contacting the lysate with an antibody specific for the extracellular domain of the human neu gene encoded p185 protein under conditions suitable for binding; and

(c) comparing the level of binding with the level of binding in a lysate of normal cells, wherein a higher level of binding is indicative of the overexpression of the human neu gene encoded p185 protein in the sample of cells.

2. The immunoassay of claim 1, wherein the sample of cells is from human breast tissue, preneoplastic tissue or neoplastic tissue.

3. The immunoassay of claim 1, wherein the antibody in step (b) is a monoclonal antibody or a polyclonal antibody.

4. An immunoassay for human neu gene encoded p185 protein in a cell lysate comprising;

(a) contacting a sample of the cell lysate with a first antibody that is specific for the extracellular domain of the human neu gene encoded p185 protein to form a first antibody-p185 protein complex.

(b) contacting the first antibody-p185 protein complex with a second antibody that is specific for a different epitope of the human neu gene encoded p185 protein to form a first antibody-p185 protein-second antibody complex; and

(c) detecting the amount of the first antibody-p185 protein-second antibody complex, wherein the amount of the first antibody-p185 protein-second antibody complex is indicative of the quantity of human neu gene encoded p185 protein in the sample.

5. An immunoassay according to claim 4 wherein the first antibody is bound to a solid support.

6. The immunoassay of claim 4 or claim 5 wherein the cell lysate is a lysate of cells from normal tissue, preneoplastic tissue or neoplastic tissue.

7. The immunoassay of any one of claims 4-6 wherein the first and/or the second antibody is a monoclonal antibody.

8. The immunoassay of claim 7 wherein the first or second antibody is a polyclonal antibody.

9. The immunoassay of any one of claims 4-8 wherein the second antibody is labeled, e.g. with biotin or horseradish peroxidase.

10. The immunoassay of any one of claims 4-9 wherein the monoclonal antibody is selected from the monoclonal antibodies having the immunological properties of BD5-2d (ATCC HB9689).

**Patentansprüche**

1. Ein Immunoassay zum Nachweis der Überexpression des menschlichen neu Gens, kodierend pl85 Protein, in einer Zellprobe umfassend:

(a) Erhalten eines Lysats der Zellprobe;

(b) In-Kontakt-Bringen des Lysats mit einem Antikörper, der für die extrazelluläre Domäne des menschlichen neu Gens, kodierend pl85-Protein, spezifisch ist, unter Bedingungen, die zum Binden geeignet sind; und

(c) Vergleichen des Bindungsgrades mit dem Bindungsgrad in einem Lysat normaler Zellen, wobei ein höherer Bindungsgrad die Überexpression des menschlichen neu Gens, kodierend pl85 Protein, in der Zellprobe anzeigt.

2. Der Immunoassay gemäß Anspruch 1, worin die Zellprobe von menschlichem Brustgewebe, präneoplastischem Gewebe oder neoplastischem Gewebe ist.

3. Der Immunoassay gemäß Anspruch 1, worin der Antikörper aus Schritt (b) ein monoklonaler Antikörper oder ein polyklonaler Antikörper ist.

4. Ein Immunoassay für menschliches neu Gen, kodierend pl85 Protein, in einem Zellysat umfassend:

(a) In-Kontakt-Bringen des Zellysats mit einem ersten Antikörper, der für die extrazelluläre Domäne des menschlichen neu Gens, kodierend pl85-Protein, spezifisch ist, um einen ersten Antikörper - pl85 Protein Komplex zu bilden;

(b) In-Kontakt-Bringen des ersten Antikörper pl85 Protein Komplexes mit einem zweiten Antikörper, der für ein anderes Epitop des menschlichen neu Gens, kodierend pl85 Protein, spezifisch ist, um einen ersten Antikörper - pl85 Protein - zweiten Antikörper Komplex zu bilden; und

(c) Nachweisen der Menge des ersten Antikörper - pl85 Protein - zweiten Antikörper Komplexes, wobei die Menge des ersten Antikörper - pl85 Protein - zweiten Antikörper Komplexes die Quantität des menschlichen neu Gens, kodierend pl85 Protein, in der Probe anzeigt.

5. Der Immunoassay gemäß Anspruch 4, worin der erste Antikörper an einen festen Träger gebunden ist.

6. Der Immunoassay gemäß Anspruch 4 oder Anspruch 5, wobei das Zellysate ein Lysat von Zellen von normalem Gewebe, präneoplastischem Gewebe oder neoplastischem Gewebe ist.

7. Der Immunoassay gemäß einem der Ansprüche 4 bis 6, worin der erste und/oder der zweite Antikörper ein monoklonaler Antikörper ist.

8. Der Immunoassay gemäß Anspruch 7, worin der erste oder der zweite Antikörper ein polyklonaler Antikörper ist.

9. Der Immunoassay gemäß einem der Ansprüche 4 bis 8, worin der zweite Antikörper markiert ist, z.B. mit Biotin oder Meerrettich-Peroxidase.

10. Der Immunoassay gemäß einem der Ansprüche 4 bis 9, worin der monoklonale Antikörper ausgewählt ist aus den monoklonalen Antikörpern, die die immunologischen Eigenschaften von BD5-2d (ATCC HB9689) haben.

**Revendications**

1. Immunodosage pour détecter une surexpression de la protéine P 185 codée par le gène neu humain dans un échantillon cellulaire comprenant :

(a) l'obtention d'un lysat de l'échantillon cellulaire ;

(b) la mise en contact du lysat avec un anticorps spécifique pour le domaine extracellulaire de la protéine P185 codée par le gène neu humain dans des conditions appropriées à la liaison ; et

(c) la comparaison du taux de liaison avec le taux de liaison dans un lysat de cellules normales, un taux de liaison plus élevé étant indicatif de la surexpression de la protéine P 185 codée par le gène neu humain dans l'échantillon cellulaire.

2. Immunodosage selon la revendication 1 où l'échantillon cellulaire provient de tissus mammaires humains, de tissus prénéoplasiques ou de tissus néoplasiques.

3. Immunodosage selon la revendication 1, où l'anticorps dans l'étape (b) est un anticorps monoclonal ou polyclonal.

4. Immunodosage pour la protéine P185 codée par le gène neu humain dans un lysat cellulaire comprenant :

(a) la mise en contact de l'échantillon de lysat cellulaire avec un premier anticorps spécifique pour le domaine extracellulaire de la protéine P185 codée par le gène neu humain pour former un premier complexe anticorps-protéine P185 ;

(b) la mise en contact du premier complexe anticorps-protéine P185 avec un second anticorps qui est spécifique pour un épitope différent de la protéine P185 codée par le gène neu humain pour former un complexe premier anticorps-protéine P185-second anticorps ; et

(c) la détection de la quantité de complexes premier anticorps-protéine P185-second anticorps, où la quantité de complexes premier anticorps-protéine P185-second anticorps est indicative de la quantité de protéine P185 codée par le gène neu humain dans l'échantillon.

5. Immunodosage selon la revendication 4, où le premier anticorps est fixé sur un support solide.

**6.** Immunodosage selon la revendication 4 ou 5, où le lysat cellulaire est un lysat de cellules provenant de tissu normal, de tissu prénéoplasique et de tissu néoplasique.

**7.** Immunodosage selon l'une quelconque des revendications 4 à 6, où le premier anticorps et/ou le second anticorps est un anticorps monoclonal.

**8.** Immunodosage selon la revendication 7, où le premier ou le second anticorps est un anticorps polyclonal.

**9.** Immunodosage selon l'une quelconque des revendications 4 à 8, où le second anticorps est marqué par exemple avec de la biotine ou de la peroxydase de raifort.

**10.** Immunodosage selon l'une quelconque des revendications 4 à 9, où l'anticorps monoclonal est choisi parmi les anticorps monoclonaux ayant les propriétés immunologiques de BD5-2d déposé à l'ATCC sous le numéro HB 9689.

# FIG.1

neu cDNA

pSV2 neu
9.6kb

SV40
Early
Promotor

Hind III

Pvu II

PBR amp R

Sal I
SV40 t Splice

SV40 Poly A

EcoR I

FIG.2B

FIG.2A

p185—

# FIG.3

```
normal                                                      val
                                                            GTG
glu gln arg ala ser pro val thr phe ile ile ala thr val  :  gly val      aa 666
GAG CAG AGA GCC AGC CCG GTG ACA TTC ATC ATT GCA ACT GTA  :  GGC GTC
                                                            GAG
transforming                                                glu


leu leu phe leu ile leu val val val val gly ile leu ile lys arg arg      aa 683
CTG CTG TTC CTG ATC TTA GTG GTG GTC GTT GGA ATC CTA ATC AAA CGA AGG
```

# FIG.4

A) ACGCCCACTACAGTTGCAAT     nucleotides 1999-2018, wild-type sequence

       *
B) ACGCCCTCTACAGTTGCAAT     nucleotides 999-2018, $T_{2012}$ to A

C) CCGTCCTCAGCTGTGACC     nucleotides 996-1013, control probe

       *
D) ACGCCCCCTACAGTTGCAAT     nucleotides 1999-2018, $T_{2012}$ to G

# FIG.5

# FIG. 6

# FIG.7

N G B 1 2 3 4 5 6 7 8 9 10 11 12 13 14 cl c2

N G B 1 2 3 4 5 6 7 8 9 10 11 12 13 14 cl c2

*Fig. 8*

Construction of pMax neu and pMax delta neu

*Fig. 9*

Neu Oncogene Probe Design

*Fig. 10*

*Fig. 11*

NEU CAPTURE ELISA USING MAB TA-1

O.D. 490nm

μg OF EXTRACT

SK-BR3
HSO578T
T47D
ZR-75-1
MCF-7

Detector AB is biotinylated NA3

Fig. 12

EP 0 412 116 B1

NEU CAPTURE ELISA USING MAB NB3

X-3-5 Nude tumor extract

3T3-RAS Nude tumor extract

Detector AB is Biotin labeled TA-1

*Fig. 13*

NEU CAPTURE OF HUMAN BREAST TISSUE

Normal breast 2747-OI-O5O
Breast tumor 2747-OI-O5O

Detector AB is Biotin labeled BD-5

*Fig. 14*

EP 0 412 116 B1

NEU CAPTURE OF CULTURE SUPERNATANTS USING MAB NB3

O.D. 490nm

1/DILUTION OF SUPERNATANT

——●—— I8-3-7
— ■ — 3T3-RAS
——△—— SKBR3 LOT #2
——◇—— DHEN IO% FCS

Detector AB is biotinylated TA-1

*Fig.* 15

EP 0 412 116 B1

NEU CAPTURE USING MAB TA-1

O.D. 490nm

1/DILUTION OF NUDE MOUSE SERUM

—□— 3T3 (RAS) Mouse
—●— 18-3-7 Mouse
—▲— T144 Mouse

Detector AB is Biotinylated BD-5

*Fig. 16*

EP 0 412 116 B1

NEU CAPTURE OF HUMAN PLASMA USING MAB TA-1

O.D. 490nm

1/ DILUTION OF PLASMA

———●——— Normal plasma
— —◇— — Patient PSUL 6/23
———□——— Patient AJAC 6/23

Detector AB is biotin labeled BD-5

*Fig.* 17

EP 0 412 116 B1

Fig. 18